# EUROPEAN PATENT APPLICATION

(11) **EP 3 367 322 A1**
(43) Date of publication of application: **29.08.2018**
(21) Application number: 16872989.5
(22) Date of filing: 06.12.2016
(51) Int. Cl.: G06Q 50/22, A61G 7/05, G08B 21/02, G08B 25/04

(54) **CENTRAL PROCESSING DEVICE AND CENTRAL PROCESSING METHOD FOR MONITORED-PERSON MONITORING SYSTEM, AND MONITORED-PERSON MONITORING SYSTEM**

(30) Priority: 09.12.2015 JP 2015239917
(71) Applicant: Konica Minolta, Inc., Tokyo 100-7015 (JP)
(72) Inventor: NISHIKADO, Masashi, Tokyo 100-7015 (JP); ATARASHI, Yuichi, Tokyo 100-7015 (JP)
(74) Representative: Hoffmann Eitle
(86) International application number: PCT/JP2016/086278
(87) International publication number: WO 2017/099091

(57) **Abstract**

In a central processing device and a central processing method for use in a subject observation system and a subject observation system according to the present invention, a care name indicating a name of a care service, a care timing indicating a timing of performing the care service and a sensor identifier possessed by the sensor unit corresponding to a subject as a target who receives the care service are stored while being associated with each other, and the care name associated with the care timing is notified together with an image acquired from the sensor unit having the sensor identifier associated with the care timing to a predetermined terminal device in accordance with the care timing.

## Description

### TECHNICAL FIELD

The present invention relates to a central processing device and a central processing method in a subject observation system for observing a subject and the subject observation system.

### BACKGROUND ART

Our country, Japan, has been experiencing an aging society, particularly, a super aging society where an aging rate of the population aged 65 and older to the total population exceeds 21 % because of improvements in living standards, sanitary conditions and medical levels accompanying with the rapid economic growth after the war. Also, the population aged 65 and older is expected to reach approximately 34,560,000 to the total population of 124,110,000 in 2020, while the population aged 65 and older was approximately 25,560,000 to the total population of approximately 127,650,000 in 2005. This aging society is expected to have a greater number of nursing or care needers (nursing needers or the like) due to illnesses, injuries or aging than a non-aging society. Moreover, our country also has been experiencing a declining-birth rate society, for example, the total fertility rate was 1.43 in 2013. This circumstance has also caused a problem of "care of an elderly person by another elderly person", which means that an elderly person who requires nursing or the like has to be taken care of by an elderly family member such as a spouse, child, or sibling.

The nursing needers or the like enter hospitals or facilities like welfare facilities (including short-stay facilities, care homes, intensive care homes, and the like referred by Japanese statutory laws) for the elderly, and receive nursing or care. These facilities face risks that nursing needers or the like get injuries by falling down from beds or falling over during walking, or loiter after sneaking out of the beds. It is necessary to eliminate the risks as soon as possible. The risks may lead to more serious problems if being left unattended without any countermeasures. Thus, nurses, caregivers or the like confirm the safety or check the state of each of the nursing needers or the like through regular patrols.

However, the nursing and care industries encounter a problem of chronic labor shortage due to a slower increase in the number of nurses or the like than in the number of nursing needers or the like. Furthermore, compared with the day time, a workload per nurse or caregiver is much heavier during the semi-night time and the night time because of a decrease in the number of nurses or caregivers during those times. Hence, there has been a demand of reduction in the workload. Moreover, the aforementioned problem of "care of an elderly person by another elderly person" is seen in the facilities as well without exception, i.e., it is often recognized that an elderly nurse or the like has to take care of an elderly care needer or the like. Generally, as one gets older, his or her physical strength declines. This means that the nursing workload is harder for an older nurse than for a younger nurse regardless of his or her good health, and the older nurse is considered to delay in movement or judgment.

In order to alleviate the labor shortage and the workload of the nurses or the like, technologies of supporting the nursing and caring workloads have been demanded. In response to this demand, subject observation apparatuses have been recently researched and developed to observe (monitor) a subject as a watching target, such as a nursing needer or the like, to be observed.

The technologies involve an exemplary call system disclosed in Patent Literature 1. The call system disclosed in Patent Literature 1 includes a subsidiary nurse call device allotted to a bed for allowing a patient to call a nurse, and a host nurse call device arranged in a nurse station for answering to the calling from the subsidiary nurse call device, and further has a camera for photographing the patient on the bed from a higher position than the bed, and state judging means for judging an occurrence of at least one of the states of the patient, i.e., raising his/her upper body and leaving away from the bed, thereby outputting a caution state occurrence signal. The host nurse call device has notification means for performing a notification upon receipt of the caution state occurrence signal. Also, the nurse call system further includes a mobile terminal carried by the nurse in order to answer to the calling from the subsidiary nurse call device, and communication control means for sending a video image photographed by the camera to the mobile terminal upon receipt of the caution state occurrence signal.

Meanwhile, a person living alone is also a subject to be observed as well as the nursing needer or the like in terms of safety confirmation.

The call system disclosed in Patent Literature 1 can receive a nurse call by the patient and the caution state occurrence signal generated according to the movement of the patient by the call system. Thus, an observer (user) such as a nurse or caregiver can give nursing or take care corresponding to the calling from the patient (subject) or the movement of the patient. However, actual nursing services, care services and the like include not only services for answering to the calling and the like as described above, but also various types of cares (care services) such as meal assistance, dressing assistance, assistance of water intake, medicine-taking, excretion and the like. Thus, the observer such as a nurse needs to memorize names of the care services (care names), timings of the cares (care timings) and contents of the cares (care contents) for the subject such as the nursing needer who is taken care of by the observer. However, the nursing industry and the like are experiencing a serious situation such as chronic labor shortage. Thus, a relatively large number of subjects are taken care of by one observer, and it is difficult for the observer to memorize the care names and the care timings of the care services for each subject, which burdens the observer. Particularly, in medicine-taking, the type and amount of medicine and a medication interval need to be delicately handled.

On the other hand, there are cases where the observer cannot perform the intended care service such as when the subject is absent or asleep when the care timing of the care service is reached and the observer prepares the care service and goes to the subject. The observer goes for nothing and cannot perform the efficient care service.

### CITATION LIST

### PATENT LITERATURE

Patent Literature 1: Japanese Unexamined Patent Publication No. 2014-90913

### SUMMARY OF INVENTION

The present invention has been accomplished in view of the above-described situations. An object of the present invention is to provide a central processing device and a central processing method for use in a subject observation system and a subject observation system capable of promoting timely and suitable execution of care services and assisting (supporting) more efficient care services.

In a central processing device and a central processing method for use in a subject observation system and a subject observation system according to the present invention, a care name indicating a name of a care service, a care timing indicating a timing of performing the care service and a sensor identifier possessed by the sensor unit corresponding to a subject as a target who receives the care service are stored while being associated with each other, and the care name associated with the care timing is notified together with an image acquired from the sensor unit having the sensor identifier associated with the care timing to a predetermined terminal device in accordance with the care timing.

These and other subjects, features and advantages of the present invention will become more apparent upon reading the following detailed description along with the accompanying drawings.

### BRIEF DESCRIPTION OF DRAWINGS

FIG. 1 shows a configuration of a subject observation system according to an embodiment.
FIG. 2 shows a configuration of a sensor unit in the subject observation system.
FIG. 3 shows a configuration of an administration server in the subject observation system.
FIG. 4 shows a configuration of a care information table stored in the administration server.
FIG. 5 shows a configuration of a sensor information table stored in the administration server.
FIG. 6 shows a configuration of a mobile terminal device in the subject observation system.
FIG. 7 is a flowchart showing an operation of the mobile terminal device for a care notification in the subject observation system.
FIG. 8 is a flowchart showing an operation of the administration server for receipt of a communication signal in the subject observation system.
FIG. 9 is a flowchart showing an operation of the mobile terminal device in the subject observation system,
FIG. 10 shows an example of a standby screen image displayed on the mobile terminal device in the subject observation system.
FIG. 11 shows an example of an observational information screen image displayed on the mobile terminal device in the subject observation system.
FIG. 12 shows an example of a care notification screen image displayed on the mobile terminal device in the subject observation system.

### DESCRIPTION OF EMBODIMENT

Hereinafter, an embodiment of the present invention will be described with reference to the accompanying drawings. Elements denoted by the same reference numerals in the drawings have the same configuration and, therefore, repeated descriptions will be appropriately omitted. In the present specification, elements are denoted by a same reference numeral when being referred to collectively, and are denoted by a same reference numeral accompanied by a different respective reference character when being referred to individually.

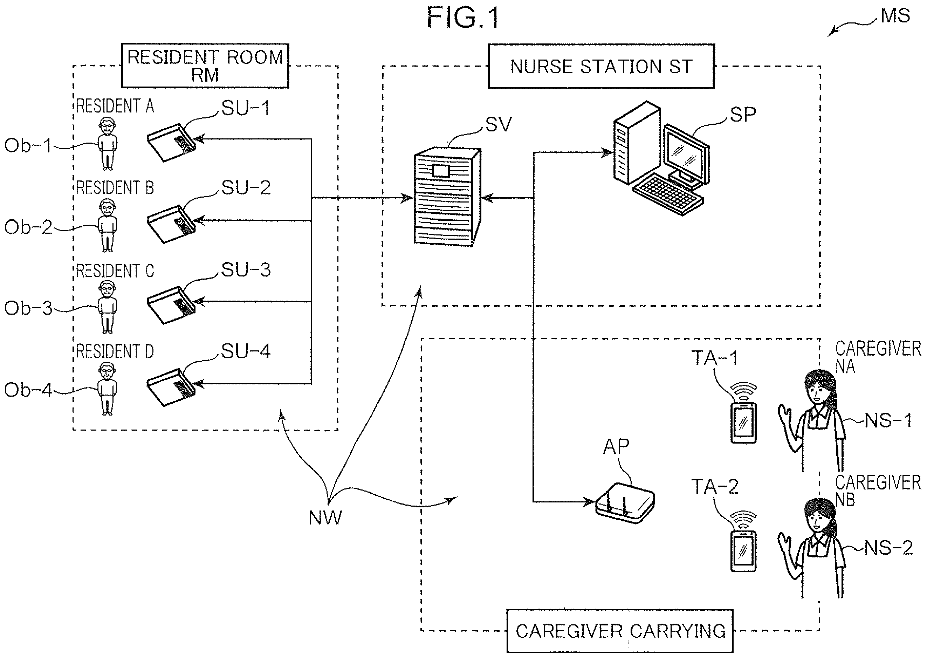

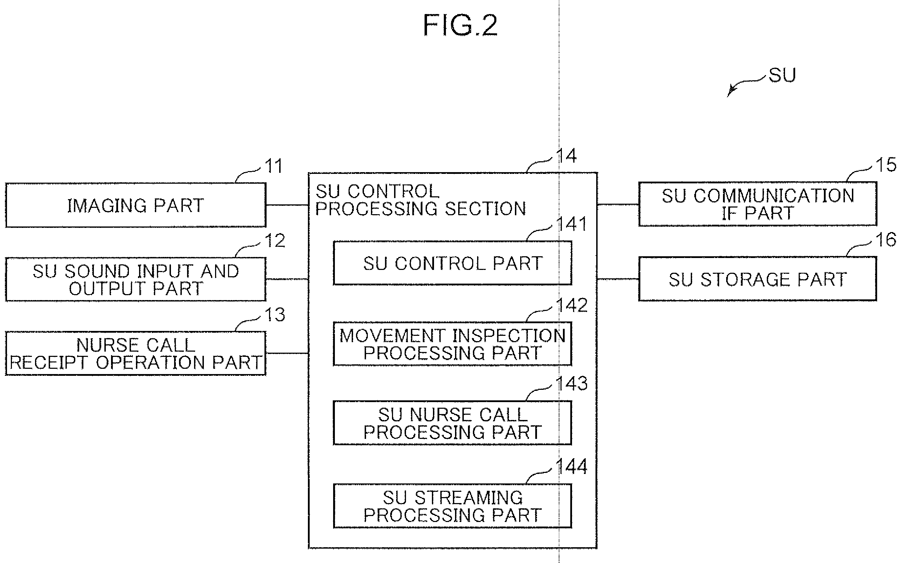

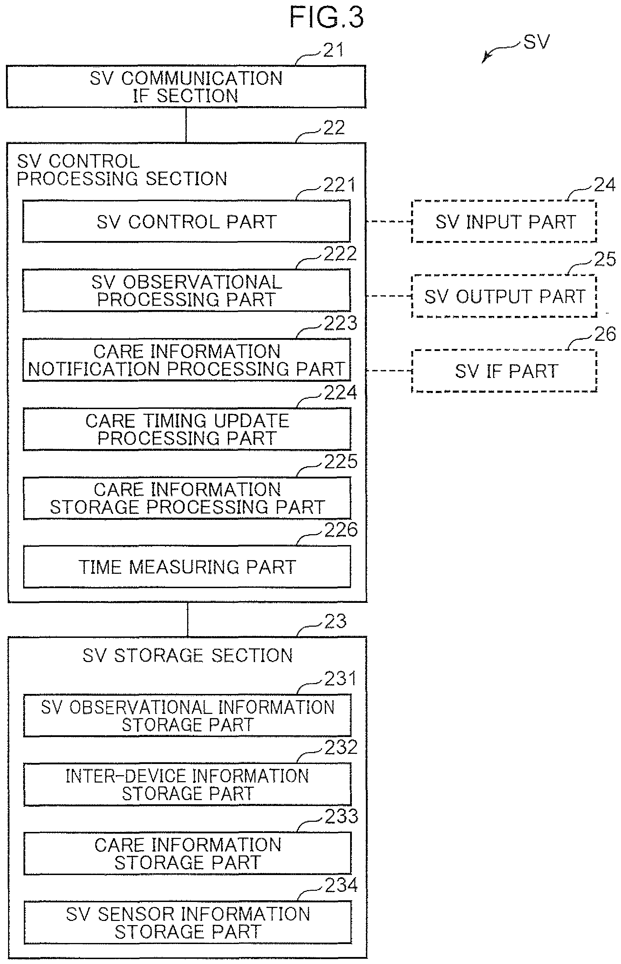

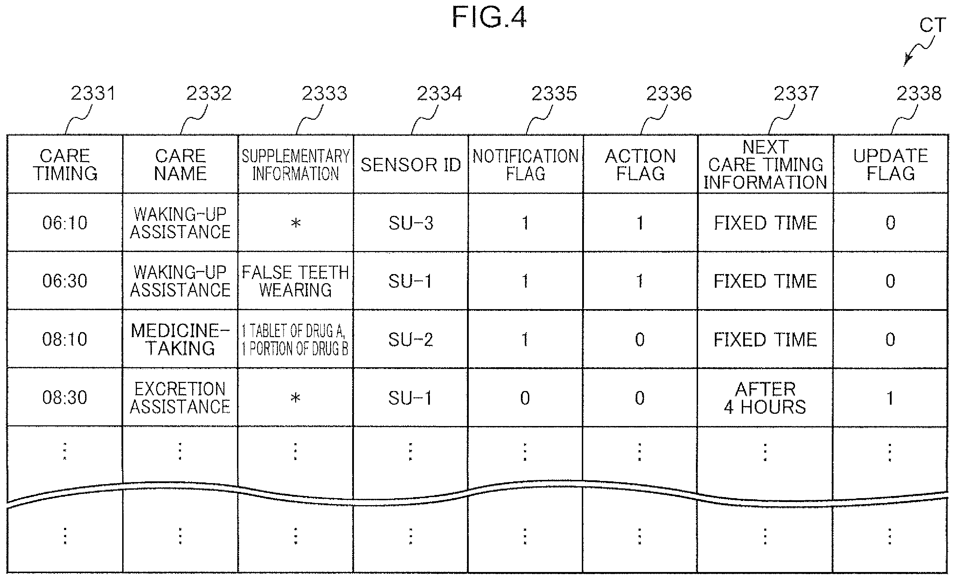

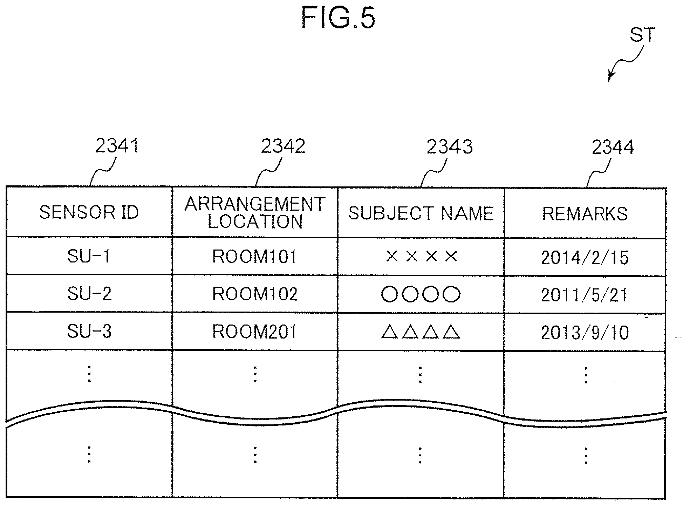

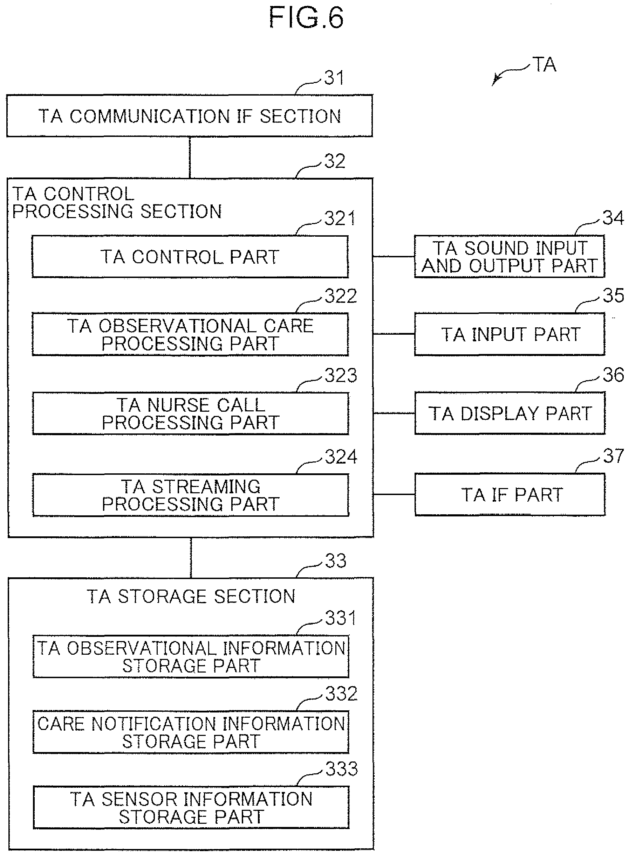

FIG. 1 shows a configuration of a subject observation system in the embodiment. FIG. 2 shows a configuration of a sensor unit in the subject observation system of the embodiment. FIG. 3 shows a configuration of an administration server in the subject observation system of the embodiment. FIG. 4 shows a configuration of a care information table stored in the administration server. FIG. 5 shows a configuration of a sensor information table stored in the administration server. FIG. 6 shows a configuration of a mobile terminal device in the subject observation system of the embodiment.

The subject observation system in the embodiment observes a subject Ob as a watching target (supervising target) to be observed (supervised) using a plurality of devices and includes sensor units, a central processing device and terminal devices. The sensor unit inspects a predetermined movement of the subject Ob set in advance based on an image and notifies an inspection result thereof to the terminal device via the central processing device. The central processing device includes a care information storage part for storing a care name indicating a name of a care service, a care timing indicating a timing of performing the care service and a sensor identifier possessed by the sensor unit corresponding to a subject as a target who receives the care service as care information while associating them with each other, an image acquiring part for acquiring an image from the sensor unit and a care information notification processing part for notifying the care name associated with the care timing together with an image acquired by the image acquiring part from the sensor unit having the sensor identifier associated with the care timing to a predetermined terminal device in accordance with the care timing stored in the care information storage part. Preferably, the central processing device further includes a sensor information storage part for storing the sensor identifier and a name of the subject as sensor information while associating them with each other, and the care information notification processing part notifies the care name associated with the care timing and the subject name associated with the sensor identifier stored in the sensor information storage part together with an image acquired by the image acquiring part from the sensor unit having the sensor identifier associated with the care timing to the predetermined terminal device in accordance with the care timing stored in the care information storage part.

Specifically, for example, as shown in FIG. 1, the subject observation system MS includes one or more sensor units SU (SU-1 to SU-4), an administration server SV, a stationary terminal device SP and one or more mobile terminal devices TA (TA-1, TA-2), which are communicatively connected with one another via a wired or wireless network NW or communication line, such as a LAN (Local Area Network), a telephone network and a data communication network. The network NW may include a relay device, such as a repeater, bridge, rooter or cross connect, to relay a communication signal. In the embodiment shown in FIG. 1, these sensor units SU-1 to SU-4, the administration server SV, the stationary terminal device SP and the mobile terminal devices TA-1, T1-2 are communicatively connected with one another via a wireless LAN (such as a LAN satisfying IEEE802.11 standard) NW including an access point AP.

As clearly seen from the detailed description below, the administration server SV is equivalent to an example of the central processing device, the stationary terminal device SP is equivalent to an example of the terminal device and the mobile terminal device TA is equivalent to another example of the terminal device. A main difference between these stationary terminal device SP and mobile terminal device TA is that the stationary terminal device SP is stationary used, whereas the mobile terminal device TA is used by being carried by an observer (user) such as a nurse or caregiver. Since these stationary terminal device SP and mobile terminal device TA are substantially similar, the mobile terminal device TA is mainly described in the embodiment.

The subject observation system MS is disposed at a location suitable for the subject Ob. The subject (supervising target) Ob may be, for example, a person who requires nursing due to an illness or injury, a person who needs care due to reduction in the physical ability, and a person living alone. Particularly, the subject Ob is appreciated to require finding of a predetermined inconvenient incident, for example, an abnormality condition, happened to the subject in terms of achievement in early finding and quick action. For this reason, the subject observation system MS is preferably disposed in a building, such as a hospital, a welfare facility for the elderly and a house, depending on a type of the subject Ob. In the embodiment shown in FIG. 1, the subject observation system MS is disposed in a care facility building provided with a plurality of chambers including resident rooms RM respectively for a plurality of subjects Ob to live therein, a nurse station, and other rooms.

Each of the sensor units SU has a communication function to communicate with the other devices SV, SP, TA via the network NW, and serves as a device for inspecting a predetermined movement of the subject Ob based on an image and notifying an inspection result to the administration server SV together with the image, receiving a nurse call and notifying that to the administration server SV, performing a voice communication with the terminal devices SP, TA, and generating images including video images and distributing the video images to the terminal devices SP, TA. The sensor unit SU includes, for example, as shown in FIG. 2, an imaging part 11, a sensor sound input and output section (SU sound input and output part) 12, a nurse call receipt operation part 13, a sensor control processing section (SU control processing part) 14, a sensor communication interface part (SU communication IF part) 15 and a sensor storage part (SU storage part) 16.

The imaging part 11 is connected to the SU control processing section 14 and serves as a device for generating images (image data) in accordance with a control of the SU control processing section 14. The images include still images (still image data) and video images (video image data). The imaging part 11 is arranged to be able to observe a space where the subject Ob is planned to be present (whereabouts space, resident room RM at an arrangement location in the embodiment shown in FIG. 1), images the whereabouts space as an imaging target from an upper position of this space, generates an image (image data) showing the imaging target viewed from above and outputs the image of the imaging target to the SU control processing section 14. Preferably, the imaging part 11 is disposed to be able to image the imaging target from a position right above a head planned position (normally, disposed position of a pillow) set in advance where the head of the subject Ob is planned to be located on a sleeping equipment (e.g. bed) on which the subject Ob is lying. The sensor unit SU acquires an image obtained by imaging the subject Ob from a position above the subject Ob, preferably an image imaged from a position right above the head planned position.

The imaging part 11 may be a device for generating images of visible light, but is a device for generating images of infrared rays in the embodiment so that the subject Ob can be monitored even in relative darkness. The imaging part 11 is, for example, a digital infrared camera with a focusing optical system for focusing an infrared optical image of an imaging target on a predetermined focusing plane, an image sensor arranged such that a light receiving surface coincides with the focusing plane and configured to convert the infrared optical image of the imaging target into an electric signal and an image processing part for generating image data as data representing the infrared image of the imaging target by image-processing an output of the image sensor in the embodiment. In the embodiment, the focusing optical system of the imaging part 11 is preferably a wide-angle optical system (so-called wide-angle lens (including a fisheye lens) having an angle of view capable of imaging the entire resident room RM where the focusing optical system is disposed.

The SU sound input and output part 12 is connected to the SU control processing section 14 and serves as a circuit for acquiring an external sound and inputting the sound to the sensor unit SU and also as a circuit for generating and outputting a sound corresponding to an electric signal representing the sound in accordance with a control of the SU control processing section 14. The SU sound input and output part 12 includes, for example, a microphone to convert a sound acoustic vibration to an electric signal, and a speaker to convert a sound electric signal to a sound acoustic vibration, and other elements. The SU sound input and output part 12 outputs an external sound representing electric signal to the SU control processing section 14 and converts an electric signal input from the SU control processing section 14 into a sound acoustic vibration.

The nurse call receipt operation part 13 is connected to the SU control processing section 14 and serves as a switch circuit such as a push-button switch for inputting a nurse call to the sensor unit SU. The nurse call receipt operation part 13 may be connected to the SU control processing section 14 by wire or may be connected to the SU control processing section 14 by short-range wireless communication such as Bluetooth (Registered Trademark).

The SU communication IF part 15 is connected to the SU control processing section 14 and serves as a communication circuit for performing a communication in accordance with a control of the SU control processing section 14. The SU communication IF part 15 generates a communication signal containing data input from the SU control processing section 14 for transfer in accordance with a communication protocol used in the network NW of the subject observation system MS, and sends this generated communication signal to the other device SV, SP, TA via the network NW. The SU communication IF part 15 receives a communication signal from the other device SV, SP, TA via the network NW, takes data from the received communication signal, converts the taken data into another data having a different format that can be processed by the SU control processing section 14, and outputs the converted data to the SU control processing section 14. The SU communication IF part 15 includes, for example, a communication interface circuit satisfying IEEE802.11 standard or the like.

The SV storage part 16 is connected to the SV control processing section 14, and serves as a circuit for storing various predetermined programs and data in accordance with a control of the SU control processing section 14. For example, the various predetermined programs include control processing programs such as a SU control program for controlling respective parts of the sensor unit SU according to their functions, and a SU observational processing program for executing a predetermined information processing about the observation of the subject Ob. The SU observational processing program includes a movement inspection processing program for inspecting a predetermined movement of the subject Ob based on an image and notifying an inspection result together with the image to the administration server SV, a SU nurse call processing program for notifying to the administration server SV upon receipt of a nurse call by the nurse call receipt operation part 13 and performing a voice communication with the terminal device SP, TA using the SU sound input and output part 12, and a SU streaming program for distributing video images generated by the imaging part 11 to the terminal device SP, TA having requested those video images by means of streaming. Various predetermined data includes data necessary for executing the respective programs, such as a sensor unit identifier (sensor ID) of the sensor unit SU for specifying and identifying the sensor unit SU and a communication address of the administration server SV. The SU storage part 16 includes, for example, a ROM (Read Only Memory), which is a nonvolatile storage element, an EEPROM (Electrically Erasable Programmable Read Only Memory), which is a rewritable nonvolatile storage element, and the like. The SU storage part 16 includes a RAM (Random Access Memory) serving as a so-called working memory of the SU control processing section 14 for storing data and the like generated during the execution of the predetermined program, and the like.

The SU control processing section 14 serves as a circuit for controlling the respective parts of the sensor unit SU according to their functions, inspecting a predetermined movement of the subject Ob based on an image, notifying an inspection result together with the image to the administration server SV, notifying to the administration server SV upon receipt of a nurse call, performing a voice communication with the terminal device SP, TA, generating images including video images and distributing the video images to the terminal device SP, TA. The SU control processing section 14 includes, for example, a CPU (Central Processing Unit) and its peripheral circuits. The SU control processing section 14 is operably provided with a sensor control part (SU control part) 141, a movement inspection processing part 142, a sensor nurse call processing part (SU nurse call processing part) 143 and a sensor streaming processing part (SU streaming processing part) 144 by executing the control processing programs.

The SU control part 141 controls the respective parts of the sensor unit SU according to their functions and controls the entire sensor unit SU.

The movement inspection processing part 142 inspects a predetermined movement of the subject Ob set in advance based on an image and notifies an inspection result together with the image to the administration server SV. Specifically, in the embodiment, examples of the predetermined movements are four movements including a waking-up movement of the subject Ob, a leaving movement of the subject Ob from a bed, a falling-down movement of the subject Ob from sleeping equipment and a falling-over movement of the subject Ob. The movement inspection processing part 142 detects the head of the subject Ob based on a target image imaged by the imaging part 11 and inspects the waking up movement, the bed-leaving movement, the fall-over movement or the falling-down movement of the subject Ob based on a change of the size of the detected head of the subject Ob over time. Specifically, a whereabouts region of a sleeping equipment BT and first to third threshold values Th1 to Th3 are stored in advance in the SU storage part 16 as one of the various predetermined data. The first threshold value Th1 is a threshold value for discriminating the size of the head in a lying posture and the size of the head in a sitting posture in the whereabouts region of the sleeping equipment BT. The second threshold value Th2 is a threshold value for discriminating whether or not the size of the head is the size of the head in a standing posture in the resident room RM excluding the whereabouts region of the sleeping equipment BT. The third threshold value Th3 is a threshold value for discriminating whether or not the size of the head is the size of the head in the lying posture in the resident room RM excluding the whereabouts area of the sleeping equipment BT. The movement inspection processing part 142 first extracts a moving body region as a human body region of the subject Ob from a target image, for example, by a background differential method or frame differential method. Subsequently, the movement inspection processing part 142 extracts a head region of the subject Ob, for example, by a circular or elliptical Hough transform, by pattern matching using a model of the head prepared in advance or by a neural network learned for head detection. Then, the movement inspection processing part 142 detects the waking-up movement, the bed-leaving movement, the fall-over movement or the falling-down movement from the extracted head position and size. For example, the movement inspection processing part 142 determines and inspects the waking-up movement if the extracted head position is in the whereabouts region of the sleeping equipment BT and the detected size of the head changes from the size in the lying posture to the size in the sitting posture over time using the first threshold value Th1. For example, the movement inspection processing part 142 determines and inspects the bed-leaving movement if the extracted head position changes from the whereabouts region of the sleeping equipment BT to the outside of the whereabouts region of the sleeping equipment BT and the size of the extracted head changes from a certain size to the size in the standing posture using the second threshold value Th2. For example, the movement inspection processing part 142 determines and inspects the fall-down movement if the extracted head position changes from the whereabouts region of the sleeping equipment BT to the outside of the whereabouts region of the sleeping equipment BT and the size of the extracted head changes from a certain size to the size in the lying posture using the third threshold value Th3. For example, the movement inspection processing part 142 determines and inspects the falling-over movement if the extracted head position is in the resident room RM excluding the whereabouts region of the sleeping equipment BT and the size of the extracted head changes from a certain size to the size in the lying posture using the third threshold value Th1. When the predetermined movement is inspected in this way, the movement inspection processing part 142 notifies this inspection result and the target image used in obtaining this inspection result to the administration server SV by means of the SU communication IF part 15. Specifically, the movement inspection processing part 142 sends a communication signal containing the sensor ID of the corresponding sensor unit SU, the inspection result (one or more of the waking-up, bed-leaving, falling-down and falling-over movements in the embodiment), the target image used for the inspection of the predetermined movement (first observational information communication signal) to the administration server SV via the SU communication IF part 15. The image may be at least one of a still image and a video image. In the embodiment, a still image is first notified and a video image is distributed according to the user's request. It should be noted that a video image may be first distributed or a still image and a video image may be sent and displayed on the terminal device SP, TA by screen splitting.

The SU nurse call processing part 143 notifies to the administration server SV upon receipt of a nurse call by the nurse call receipt operation part 13 and performs a voice communication with the terminal device SP, TA using the SU sound input and output part 12 and the like. Specifically, when the nurse call receipt operation part 13 is operated for input, the SU nurse call processing part 143 sends a communication signal containing nurse call receipt information representing the receipt of a nurse call and the sensor ID of the sensor unit SU (nurse call notification communication signal) to the administration server SV via the SU communication IF part 15. Then, the SU nurse call processing part 143 performs a voice communication with the terminal device SP, TA, for example, by VoIP (Voice over Internet Protocol) using the SU sound input and output part 12 and the like.

If a request to distribute video images is made from the stationary terminal device SP or mobile terminal device TA via the communication IF section 3, the SU streaming processing part 144 distributes video images (e.g. live video images) generated by the imaging part 11 to the statuary terminal device SP or mobile terminal device TA having made this request via the SU communication IF part 15 by means of streaming reproduction.

Four first to fourth sensor units SU-1 to SU-4 are exemplarily shown in FIG. 1. The first sensor unit SU-1 is disposed in a resident room RM-1 (not shown) of a resident A Ob-1 as one of the subjects Ob, the second sensor unit SU-2 is disposed in a resident room RM-2 (not shown) of a resident B Ob-2 as one of the subjects Ob, the third sensor unit SU-3 is disposed in a resident room RM-3 (not shown) of a resident C Ob-3 as one of the subjects Ob and the fourth sensor unit SU-4 is disposed in a resident room RM-4 (not shown) of a resident D Ob-4 as one of the subjects Ob.

The administration server SV has a communication function and the like to communicate with the other devices SU, SP, TA via the network NW, and serves as a device for managing information about the observation of the subject Ob (observational information) upon receipt of the notification of the inspection result from the sensor unit SU, transmitting (retransmitting, transferring, sending) the inspection result to a predetermined terminal device SP, TA, providing data corresponding to a request of a client (terminal device SP, TA or the like in the embodiment) to the client and managing the entire subject observation system MS. The administration server SV in the embodiment notifies the care name associated with the care timing and, further supplementary information in the embodiment, together with an image acquired by a SV communication IF section 21 to be described later from the sensor unit SU (sensor unit SU sensing the subject Ob as a target who receives the care service having the care name) having the sensor ID associated with the care timing to the predetermined terminal device SP, TA in accordance with the care timing. As shown in FIG. 3, the administration server SV includes, for example, the server communication interface section (SV communication IF section) 21, a server control processing section (SV control processing section) 22, and a server storage section (SV storage section) 23.

Similarly to the SU communication IF part 15, the SV communication IF section 21 is connected to the SV control processing section 22, and serves as a circuit for performing a communication in accordance with a control of the SV control processing section 22. The SV communication IF section 21 includes a communication interface circuit satisfying, for example, the IEEE 802.11 standard.

The SV storage section 23 is connected to the SV control processing section 22, and serves as a circuit for storing various predetermined programs and data in accordance with a control of the SV control processing section 22. For example, the various predetermined programs include control processing programs such as a SV control program for controlling respective parts of the administration server SV in accordance with their functions, and a SV observational processing program for executing a predetermined information processing about the observation of the subject Ob. The SV observational processing program includes a SV observational processing program for controlling the observational information about the observation of the subject Ob upon receipt of the notification of the inspection result from the sensor unit SU and notifying the inspection result to the predetermined terminal device SP, TA, a care information notification processing program for notifying the care name associated with the care timing together with an image acquired by the SV communication IF section 21 from the sensor unit SU having the sensor ID associated with the care timing to the predetermined terminal device SP, TA in accordance with the care timing stored in a care information storage part 233 to be described later, a care timing update processing program for updating the care timing stored in the care information storage part 233 to a next care timing when the care name is notified together with the image to the predetermined terminal device SP, TA by the care information notification processing program, a care information storage processing program for storing received execution intension information in the care information storage section 233 while further associating this information with the care name upon receipt of the execution intension information representing an intention to perform the care service corresponding to the care name notified from the administration server SV by the care information notification processing program, a clock program for measuring time and the like. The various predetermined data includes data necessary for executing the respective programs, such as a server identifier (server ID) serving as an identifier for specifying and identifying the administration server SV, observational information about the observation of the subject Ob, inter-device information representing an association between devices SU, SP, TA such as a transmission destination of the inspection result, care information about the care services, and sensor information about the sensor units SU. To store each of these observational information, inter-device information, care information and sensor information, the SV storage section 23 is operably provided with a server observational information storage part (SV observational information storage part) 231, an inter-device information storage part 232, a care information storage part 233 and a server sensor information storage part (SV sensor information storage part) 234.

The SV observational information storage part 231 stores a sensor ID, an inspection result and an image (target image) contained in a first observational information communication signal received from the sensor unit SU and a receipt time of the first observational information communication signal and the like as the observational information while associating them with each other. In the embodiment, the SV observational information storage part 231 stores the observational information of the subject Ob sent and received to and from the respective devices SU, SP, TA about the observation of the subject Ob such as nurse call receipt information and the sensor ID contained in a nurse call notification communication signal received from the sensor unit SU and a receipt time of the nurse call notification communication signal and the like.

The inter-device information storage part 232 stores a transmission destination associative relationship, a communication address associative relationship and the like as the inter-device information in the embodiment. The transmission destination associative relationship is an associative relationship between the sensor ID as a sending source and a terminal ID as a transmission destination and represents a transmission destination (retransmission destination, transfer destination, sending destination) of the first observational information communication signal and the nurse call notification communication signal and the like sent from the sensor unit SU. The communication address associative relationship is an associative relationship between the ID (sensor ID, terminal ID) of each device SU, SP, TA and the communication address thereof. The terminal ID (terminal identifier) is an identifier for specifying and identifying the terminal device SP, TA. It should be noted that each of the sensor ID, the server ID and the terminal ID may be, for example, a serial number or the like composed of a predetermined symbol string or, for example, a communication address (in this case, the communication address associative relationship can be omitted). Further, the transmission destination associative relationship is also utilized in determining a notification destination of the care service (sending destination of the care information notification communication signal) in accordance with the care timing (the terminal ID corresponding to the sensor ID associated with the care timing is the notification destination (sending destination)).

The care information storage part 233 stores the care information. In the embodiment, the care information is information associating the care name as the name of the care service, the care timing as a timing of performing the care service and the sensor ID possessed by the sensor unit SU corresponding to the subject as a target who receives the care service with each other. In the embodiment, predetermined supplementary information utilized in performing the care service is further associated with the care timing of the care service in the care information. Examples of the care service include meal assistance for assisting a meal, dressing assistance for assisting dressing (e.g. washing, cleaning, changing of clothes), body position conversion for converting a body position, water intake for taking water, medicine-taking for causing the subject to take medicine, excretion assistance for assisting excretion, hair washing for washing hair, bathing assistance for assisting bathing, infusion exchange for exchanging an infusion and blood drawing for drawing blood. Examples of the supplementary information include procedures of the care services, cautions to be taken in performing the care services, the amount of water when the care service is water intake, the type and amount of medicine and a minimum medication interval when the care service is medicine-taking, the type of liquid, the type and amount of medicine to be added to an infusion when the care service is an infusion exchange, and blood drawing items when the care service is blood drawing.

The care information is stored in a table format in the care information storage part 233 in the embodiment. Specifically, for example, as shown in FIG. 4, a care information table CT includes a care timing field 2331 for registering the care timings, a care name field 2332 for registering the care names associated with the care timings registered in the care timing field 2331, a supplementary information field 2333 for registering the supplementary information associated with the care timings registered in the care timing field 2331 (i.e. supplementary information corresponding to the care services having the care names registered in the care name field 2332), a sensor ID field 2334 for registering the sensor IDs associated with the care timings registered in the care timing field 2331 (i.e. subjects Ob who receive the care services having the care names registered in the care name field 2332), a notification flag field 2335 for registering a notification flag indicating whether or not a notification corresponding to the care timing registered in the care timing field 2331 has been made to the predetermined terminal device SP, TA), an action flag field 2336 for registering an action flag indicating whether or not the execution intention information has been received from the predetermined terminal device SP, TA for the notification (care name and sensor ID) corresponding to the care timing registered in the care timing field 2331, a next care timing information field 2337 for registering information representing an updating method when the care timing registered in the care timing field 2331 is updated to a next care timing (next care timing information) and an update flag field 2338 for registering an update flag indicating whether or not the care timing registered in the care timing field 2331 has been updated to the next care timing, and has a record for each care timing, each care name and each sensor ID (i.e. for each individual care service for each subject Ob).

A time at which the care service should be performed is, for example, registered as the care timing in the care timing field 2331. It should be noted that the day of the week and a date may be further registered in the care timing field 2331. If there is no supplementary information associated with the care timing registered in the care timing field 2331, a predetermined code indicating the absence of the supplementary information such as "*" is registered in the supplementary information field 2333. Also in default, the predetermined code indicating the absence of the supplementary information is registered in the supplementary information field 2333. It should be noted that, if a data amount of the supplementary information exceeds a storage capacity assigned to the supplementary information field 2333, a file recording the supplementary information may be prepared and a name of this file may be registered in the supplementary information field 2333. "0" or "1" is registered as the notification flag in the notification flag field 2335. The notification flag "0" means that a notification corresponding to the care timing registered in the care timing field 2331 has not been given to the predetermined terminal device SP, TA, and the notification flag "1" means that the notification corresponding to the care timing registered in the care timing field 2331 has been (already) given to the predetermined terminal device SP, TA. "0" is registered in the notification flag field 2335 in default. The execution intention information is information representing an intention to perform the care service corresponding to the care name and the sensor ID (i.e. subject Ob) notified from the administration server SV in accordance with the care timing registered in the care timing field 2331, and received from the terminal device SP, TA by a user expressing the intention as described later. It should be noted that this execution intention information is also utilized as information representing an intention to perform a predetermined action (reply, execution) such as lifesaving, nursing, care or assistance in response to a notification in the embodiment when a predetermined movement of the subject Ob is inspected as described later. "0" or "1" is registered as the action flag in the action flag field 2336. The action flag "0" means that the execution intention information has not been received from the terminal device SP, TA for the care service corresponding to the care name and the sensor ID notified from the administration server SV in accordance with the care timing registered in the care timing field 2331, and the action flag "1" means that the execution intention information has been (already) received from the terminal device SP, TA for the care service corresponding to the care name and the sensor ID notified from the administration server SV in accordance with the care timing registered in the care timing field 2331. "0" is registered in the action flag field 2336 in default. "Fixed time" for setting the next care timing for notification substantially at the same timing every day, a time interval (e.g. "after three hours", "after ten hours") until the next notification and the like are registered as the next care timing information in the next care timing information field 2337. If there is no next care timing information registered in the next care timing information field 2337 (e.g. in the case of the care service performed only once or in the case of the care service not regularly performed), a predetermined code indicating the absence of the next care timing information such as "*" is registered in the next care timing information field 2337. Also in default, the predetermined code indicating the absence of the next care timing information is registered in the next care timing information field 2337. "0" or "1" is registered as the update flag in the update flag field 2338. The update flag "0" means that the care timing registered in the care timing field 2331 has not yet been updated, and the update flag "1" means that the care timing registered in the care timing field 2331 has been (already) updated. "0" is registered in the update flag field 2338 in default.

For example, in an example shown in FIG. 4, "06:10" is registered in the care timing field 2331, "wake-up assistance" is registered in the care name field 2332, "*" is registered in the supplementary information field 2333, "SU-3" is registered in the sensor ID field 2334, "1" is registered in the notification flag field 2335, "1" is registered in the action flag field 2336 and "fixed time" is registered in the next care timing information field 2337 in a record of the first row.

The SV sensor information storage part 234 stores the sensor information. In the embodiment, the sensor information is information about the sensor units SU and also information associating the sensor IDs of the sensor units SU and the subject names of the subjects Ob with each other.

The sensor information is stored in a table format in the SV sensor information storage part 234 in the embodiment. Specifically, for example, as shown in FIG. 5, a sensor information table ST includes a sensor ID field 2341 for registering the sensor IDs, an arrangement location field 2342 for registering the arrangement locations of the sensor units SU having the sensor IDs registered in the sensor ID field 2341, a subject name field 2343 for registering the subject name of the subject Ob observed by the sensor unit SU having the sensor ID registered in the sensor ID field 2341 (i.e. subject Ob at the arrangement location of the sensor unit SU having the sensor ID registered in the sensor ID field 2341), and a remarks field 2344 for registering remarks about the sensor units SU having the sensor IDs registered in the sensor ID field 2341, the arrangement locations of the sensor units SU and the subjects Ob at the arrangement locations, and has a record for each sensor ID (i.e. for each sensor unit SU).

For example, in an example shown in FIG. 5, "SU-1" is registered in the sensor ID field 2341, "Room 101" is registered in the arrangement location field 2342, "xxxx" is registered in the subject name field 2343 and "15/2/2014" is registered as a date of entry into the room in the remarks field 2334 in the record of the first row.

The SV control processing section 22 serves as a circuit for controlling the respective parts of the administration server SV according to their functions, managing the observational information about the observation of the subject Ob upon receipt of the notification of the inspection result from the sensor unit SU, notifying the inspection result to the predetermined terminal device SP, TA, providing data corresponding to a request of the client to the client, notifying the care name and the image in accordance with the care timing and managing the entire subject observation system MS. The SV control processing section 22 includes, for example, a CPU (Central Processing Unit) and its peripheral circuits. The SV control processing section 22 is operably provided with a server control part (SV control part) 221, a server observational processing part (SV observational processing part) 222, a care information notification processing part 223, a care timing update processing part 224, a care information storage processing part 225 and a time measuring part 226 by executing the control processing programs.

The SV control part 221 controls the respective parts of the administration server SV in accordance with their functions to thereby control the entire administration server SV.

Upon receipt of the notification of the inspection result from the sensor unit SU, the SV observational processing part 222 manages the observational information about the observation of the subject Ob and notifies the inspection result to the predetermined terminal device SP, TA. Specifically, upon receipt of the first observational information communication signal from the sensor unit SU, the SV observational processing part 222 stores (records) the observational information about the observation of the subject Ob contained in the received first observational information communication signal in the SV observational information storage part 231. The SV observational processing part 222 selects (searches) a transmission destination (retransmission destination, transfer destination, sending destination) corresponding to the sensor unit SU having sent the first observational information communication signal from the transmission destination associative relationship stored in the inter-device information storage part 232 and sends a second observational information communication signal to the selected terminal device SP, TA. This selection (searching process) is performed based on the sensor ID corresponding to the sensor unit SU having sent the received first observational information communication signal. The second observational information communication signal contains the sensor ID, the inspection result and the image contained in the first observational information communication signal and a communication address corresponding to the sensor unit SU having the sensor ID contained in the first observational information communication signal as a download destination of video images. The communication address is selected (searched) from the communication address associative relationship based on the sensor ID corresponding to the sensor unit SU having sent the received first observational information communication signal. On the other hand, upon receipt of a nurse call notification communication signal from the sensor unit SU, the SV observational processing part 222 stores (records) the observational information about the observation of the subject Ob contained in the received nurse call notification communication signal in the SV observational information storage part 231. The SV observational processing part 222 selects (searches) a transmission destination (retransmission destination, transfer destination, sending destination) corresponding to the sensor unit SU having sent the received nurse call notification communication signal from the transmission destination associative relationship stored in the inter-device information storage part 232 and transfers the nurse call notification communication signal to the selected terminal device SP, TA.

The care information notification processing part 223 notifies the care name associated with the care timing together with the image acquired by the SV communication IF section 21 from the sensor unit SU having the sensor ID associated with the care timing to the predetermined terminal device SP, TA in accordance with the care timing stored in the care information storage part 233. In the embodiment, the care information notification processing part 223 also notifies the supplementary information associated with the care timing. Specifically, the care information notification processing part 223 notifies the care name and the supplementary information associated with the care timing together with the image acquired by the communication IF section 21 from the sensor unit SU having the sensor ID associated with the care timing to the terminal device SP, TA as a transmission destination corresponding to the sensor unit SU, for example, in accordance with the care timing stored in the care information storage part 233. Specifically, the care information notification processing part 223 first reads a current time from the time measuring part 226 and selects (searches) a record, in which "0" is registered as the notification flag in the notification flag field 2335, out of records having times before the read current time as care timings registered in the care timing field 2331 in the care information table CT stored in the care information storage part 233. Subsequently, the care information notification processing part 223 reads the care name, the sensor ID and the supplementary information respectively from the care name field 2332, the sensor ID field 2334 and the supplementary information field 2333, selects (searches) a notification destination (transmission destination) corresponding to the read sensor ID from the transmission destination associative relationship stored in the inter-device information storage part 232, acquires an image from the sensor unit SU having the read sensor ID and sends a care information notification communication signal to the selected terminal device SP, TA. Then, the care information notification processing part 223 updates each of the notification flag, the action flag and the update flag by registering "1", "0" and "0" respectively in the notification flag field 2335, the action flag 2336 and the update flag field 2338 in the selected record. The care information notification communication signal contains the selected care name, sensor ID and supplementary information, the image acquired by the SV communication IF section 21 from the sensor unit SU having the sensor ID and a communication address corresponding to the sensor unit SU having the selected sensor ID as a download destination of video images. It should be noted that the subject name corresponding to the read sensor ID may be selected (searched) from the sensor information table ST stored in the SV sensor information storage part 234 and the selected subject name may be further contained in the care information notification communication signal. Further, if information similar to the above-described sensor information is stored in the terminal devices SP, TA, the subject name can be specified from the sensor ID. Thus, the subject name needs not be contained in the care information notification communication signal. As just described, the subject name and the sensor ID may be substituted for each other (i.e. the sensor ID means the subject name and the subject name means the sensor ID).

In the embodiment, after notifying the care name together with the image to the predetermined terminal device SP, TA, the care information notification processing part 223 further notifies the care name together with the image acquired by the SV communication IF section 21 from the sensor unit SU having the sensor ID corresponding to the care name via the care timing to the predetermined terminal device SP, TA if the execution intension information is not associated with the care name after the elapse of a predetermined time. Specifically, the care information notification processing part 223 first reads a current time from the time measuring part 226, for example, after the elapse of the predetermined time and selects (searches) a record, in which "1" is registered as the notification flag in the notification flag field 2335 and "0" is registered as the action flag in the action flag field 2336, out of records having times before the read current time registered as care timings in the care timing field 2331 in the care information table CT stored in the care information storage part 233. Then, the care information notification processing part 223 reads the care name, the sensor ID and the supplementary information respectively from the care name field 2332, the sensor ID field 2334 and the supplementary information field 2333 in the selected record, selects (searches) a notification destination (transmission destination) corresponding to the read sensor ID from the transmission destination associative relationship stored in the inter-device information storage part 232, acquires an image from the sensor unit SU having the read sensor ID and sends a care information notification communication signal to the selected terminal device SP, TA. In this way, the notification is resent (re-notified) together with the image at that timing for the unacted care service. Thus, a current state of the subject Ob is reflected in the image.

The care timing update processing part 224 updates the care timing stored in the care information storage part 223 to a next care timing when the care information notification processing part 223 notifies the care name together with the image to the predetermined terminal device DP, TA. Specifically, for example, after the care information notification processing part 223 transmits the care information notification communication signal to the predetermined terminal device SP, TA via the SV communication IF section 21, the care timing update processing part 224 reads a current time from the time measuring part 226 after the elapse of a predetermined time, selects (searches) a record, in which "1" is registered as the notification flag in the notification flag field 2335, "1" is registered as the action flag in the action flag field 2336 and "0" is registered as the update flag in the update flag field 2338, out of records having times before the read current time registered as care timings in the care timing field 2331 in the care information table CT stored in the care information storage part 233, and reads the care timing and next care timing information respectively from the care timing field 2331 and the next care timing information field 2337 in the selected record. Then, if the read next care timing information represents another update method excluding "fixed time" such as "time interval", the care timing update processing part 224 obtains a next care timing according to the read next care timing information, updates the care timing by registering the obtained next care timing in the care timing field 2331 in the selected record, and updates each of the notification flag, the action flag and the update flag by registering "0", "0" and "1" respectively in the notification flag field 2335, the action flag field 236 and the update flag field 2338 in the selected record. Further, every observational service day (e.g. at AM0:00 if one observational service day is defined to be from AM0:00 to AM0:00 on the following day, or AM8:00 if one observational service day is defined to be from AM8:00 to AM8:00 on the following day), the care timing update processing part 224 selects (searches) a record, in which "1" is registered as the notification flag in the notification flag field 2335, "1" is registered as the action flag in the action flag field 2336 and "0" is registered as the update flag in the update flag field 2338, out of records having "fixed time" registered in the next care timing information field 2337 in the care information table CT stored in the care information storage part 233, and updates each of the notification flag, the action flag and the update flag by registering "0", "0" and "1" respectively in the notification flag field 2335, the action flag field 2336 and the update flag field 2338 in the selected record. It should be noted that since the next care timing information is "fixed time", the care timing registered in the care timing field 2331 is substantially updated to the next care timing by registering "0", "0" and "1" respectively in the notification flag field 2335, the action flag field 2336 and the update flag field 2338 in the selected recorded even if a registered content of the care timing field 2331 is not updated.

Upon receipt of execution intention information representing an intention to perform the care service corresponding to the care name notified from the administration server SV by the care information notification processing part 223, the care information storage processing part 225 stores the received execution intention information further in association with the care name in the care information storage part 233. The sending of the execution intention information by the terminal device SP, TA is described later. The terminal device SP, TA sends a communication signal (action notification communication signal) containing the care name and the sensor ID contained in the care information communication signal and the execution intention information. Thus, in the embodiment, for example, upon receipt of the action notification communication signal from the terminal device SP, TA, the care information storage processing part 225 reads a current time from the time measuring part 226, selects (searches) a record, in which the care name and the sensor ID contained in the received action notification communication signal are respectively registered in the care name field 2332 and the sensor ID field 2334, "1" is registered as the notification flag in the notification flag field 2335 and "0" is registered as the action flag in the action flag field 2336, out of records having times before the read current time registered as care timings in the care timing field 2331 in the care information table CT stored in the care information storage part 233, and updates the action flag by registering "1" in the action flag field 236 in the selected record.

The time measuring part 226 measures time. It should be noted that the time measuring part 226 may further measure the day of the week and a date in accordance with the care timing registered in the care timing field 2331.

As shown by the dashed line in FIG. 3, the administration server SV may be appreciated to include, for example, a server input part (SV input part) 24 for inputting various commands and data, a server output part (SV output part) 25 for outputting the various commands and data input by the SV input part 24, observational information about the observation of the subject Ob and the like, and a server interface part (SV IF part) 26 for performing the input and output of the data in cooperation with the external devices, the parts 24, 25 and 26 being connected to the SV control processing section 22.

The administration server SV may be made up by, for example, a computer having a communication function.

The stationary terminal device SP has a communication function to communicate with the other devices SU, SV, TA via the network NW, a display function to display predetermined information, and an input function to input a predetermined instruction or predetermined data, and further serves as a user interface (UI) of the subject observation system MS by inputting the predetermined instruction or data to be given to the administration server SV or the mobile terminal device TA, displaying the observational information obtained at the sensor unit SU and the like. For example, the stationary terminal device SP may be made up by a computer having a communication function. Here, the stationary terminal device SP, which is a terminal device, can work in a similar manner to the mobile terminal device TA. In the specification, the terminal device will be exemplarily described with reference to the mobile terminal device TA which is a terminal device.

The mobile terminal device TA has a communication function to communicate with the other devices SV, SP, SU via the network NW, a display function to display predetermined information, an input function to input a predetermined instruction or data, and a talking function to perform a voice communication, and serves as a device for inputting the predetermined instruction or data to be given to the administration server SV or the sensor unit SU, displaying the observational information (including video images) obtained by the sensor unit SU by means of a transmission from the administration server SV, displaying the care name of the care service and an image by means of a notification from the administration server SV and answering to a nurse call or talking by means of a voice communication with the sensor unit SU. In the embodiment, as shown in FIG. 6, the mobile terminal device TA includes, for example, a terminal communication interface section (TA communication IF section) 31, a terminal control processing section (TA control processing section) 32, a terminal storage section (TA storage section) 33, a terminal sound input and output part (TA sound input and output part) 34, a terminal input part (TA input part) 35, a terminal display part (TA display part) 36, and a terminal interface part (TA IF part) 37.

The TA sound input and output part 34 is connected to the TA control processing section 32, and serves as a circuit for acquiring an external sound and inputting the acquired sound into the mobile terminal device TA, and further generating and outputting a sound corresponding to a sound representative electric signal in accordance with a control by the TA control processing section 32. Like the SU sound input and output part 12, the TA sound input and output part 34 includes, for example, a microphone to convert a sound acoustic vibration to an electric signal, a speaker to convert a sound electric signal to a sound acoustic vibration, and other elements. The TA sound input and output part 34 outputs an external sound representative electric signal to the TA control processing section 32, and converts an electric signal input from the TA control processing section 32 to a sound acoustic vibration to thereby output the converted sound acoustic vibration.

The TA input part 35 is connected to the TA control processing section 32, and serves as a circuit, such as a plurality of switches allotted with predetermined functions, for accepting a predetermined operation and inputting it into the mobile terminal device TA. The predetermined operation involves various operations necessary for the observation, for example, an operation of inputting an ID for logging in, other operations of requesting and finishing a voice communication, further other operations of requesting and finishing a live video image, and a still further another operation of inputting an intention to act to the inspection result for the notified subject Ob and an intention to perform the notified care service (execution intention information). The TA display part 36 is connected to the TA control processing section 32, and serves as a circuit, such as an LCD (Liquid Crystal Display) and an organic EL display, for displaying contents of the predetermined operation input from the TA input part 35 and the observational information about the subject Ob (for example, a classified predetermined movement having been inspected by the sensor unit SU, or an image, such as a still image or a video image, of the subject Ob) observed by the subject observation system MS, and displaying the care name of the care service notified from the administration server SV, an image, receipt of a nurse call and the like. In the embodiment, the TA input part 35 and the TA display part 36 constitute a touch panel. In this case, the TA input part 35 is a positional input device which effects an input by detecting an operated position in a resistive membrane way or electrostatic capacity way, for example. The touch panel provides the positional input device over the display screen of the TA display part 36. The TA display part 36 displays one or more inputtable candidate contents. For example, when a user (observer) such as a nurse or a caregiver touches a position displaying an input content which the user wants to input, the positional input device detects the touched position, and the content displayed at the detected position is input to the mobile terminal device TA as an input content operated by the user.

The TA IF part 37 is connected to the TA control processing section 32, and serves as a circuit for inputting and outputting data with an external device in accordance with a control of the TA control processing section 32, i.e., an interface circuit adopting the Bluetooth (Registered Trademark) standard, the IrDA standard used for infrared communication, or the USB standard, or the like.

Like the SU communication IF part 15, the TA communication IF section 31 is connected to the TA control processing section 32, and serves as a communication circuit for performing a communication in accordance with a control of the TA control processing section 32. The TA communication IF section 31 generates a communication signal containing the data input from the TA control processing section 32 for transfer in accordance with a communication protocol used in the network NW of the subject observation system MS, and sends the generated communication signal to the other devices SU, SV, SP via the network NW. The TA communication IF section 31 receives a communication signal from the devices SU, SV, SP via the network NW, takes data from the received communication signal, converts the taken data to another one having a different format that can be processed by the TA control processing section 32, and outputs the converted data to the TA control processing section 32. The TA communication IF section 31 includes, for example, a communication interface circuit satisfying the IEEE802.11 standard.

The TA storage section 33 is connected to the TA control processing section 32, and serves as a circuit for storing various predetermined programs and data in accordance with a control of the TA control processing section 32. The various predetermined programs include, for example, control processing programs such as a TA control program for controlling the respective parts of the mobile terminal device TA in accordance with their functions and a TA observational processing program for executing a predetermined information processing about the observation of the subject Ob. The TA observational processing program further involves a TA observational care processing program for storing (recording) information about the observation of the subject Ob (observational information) upon receipt of the notification of an inspection result from the administration server SV, storing (recording) information about the notified care service (care notification information) upon receipt of the notification of the care service from the administration server SV and notifying an action notification communication signal upon receipt of the execution intention information from the TA input part 35, a TA nurse call processing program for performing a voice communication with the sensor unit SU using the TA sound input and output part 12, a streaming processing program for receiving a distribution of video images from the sensor unit SU and displaying the distributed video images by means of streaming reproduction, and the like. The various predetermined data includes data necessary for executing the respective programs, such as a terminal ID of the terminal device TA, screen information to be displayed on the TA display part 36, the observational information about the observation of the subject Ob, the care notification information about the notified care service and sensor information about the sensor unit SU. The TA storage section 33 is operably provided with a terminal observational information storage part (TA observational information storage part) 331, a care information storage part 332 and a terminal sensor information storage part (TA sensor information storage part) 333 for storing the observational information, the care notification information and the sensor information respectively.

In the embodiment, the TA observational information storage part 331 stores a sensor ID, an inspection result and an image contained in a second observational information communication signal received from the administration server SV, a communication address of the sensor unit SU as a download destination of video images, and a receipt time of the second observational information communication signal and the like as the observational information while associating them with each other. In the embodiment, the TA observational information storage part 331 stores the observational information of the subject Ob sent and received to and from the respective devices SU, SV, TA about the observation of the subject Ob such as nurse call receipt information and a sensor ID contained in a nurse call notification communication signal received from the sensor unit SU via the administration server SV and a receipt time of the nurse call notification communication signal.

In the embodiment, the care notification information storage part 332 stores a care name, a sensor ID, supplementary information, an image and a communication address of the sensor unit SU as a download destination of video images contained in a care information notification communication signal received from the administration server SV, a receipt time of the care information notification communication signal and the like while associating them with each other.

The TA sensor information storage part 333 stores the sensor information. Like the SV sensor information storage part 234, the TA sensor information storage part 333 stores a sensor ID, an arrangement location, a subject name and remarks while associating them with each other.

The TA control processing section 32 is a circuit for controlling the respective parts of the mobile terminal device TA in accordance with their functions, receiving and displaying the observational information about the subject Ob, receiving and displaying the notification of the care service for the subject Ob, accepting the input of the execution intention information and sending the execution intention information to the administration server SV, answering to a nurse call and talking. The TA control processing section 32 includes, for example, a CPU and its peripheral circuits. The TA control processing section 32 is operably provided with a terminal control part (TA control part) 321, a terminal observational care processing part (TA observational care processing part) 322, a terminal nurse call processing part (TA nurse call processing part) 323 and a terminal streaming processing part (TA streaming processing part) 324 by executing the control processing program.

The TA control part 321 controls the respective parts of the mobile terminal device TA in accordance with their functions to thereby control the entire mobile terminal device TA.

The observational care processing part 322 performs a predetermined information processing about the observation of the subject Ob. Specifically, upon receipt of a second observational information communication signal from the administration server SV, the TA observational care processing part 322 stores (records) the observational information about the observation of the subject Ob in the TA observational information storage processing part 331 based on each information (each data) contained in the received second observational information communication signal. The TA observational care processing part 322 displays a screen corresponding to each information contained in the received second observational information communication signal on the TA display part 36. Upon receipt of a care information notification communication signal from the administration server SV, the TA observational care processing part 322 stores (records) the care notification information about the care service for the subject Ob based on each information (each data) contained in the received care information notification communication signal. The TA observational care processing part 322 displays a screen corresponding to each information contained in the received care information notification communication signal on the TA display part 36. Upon acceptance of a predetermined input operation from the TA input part 35, the TA observational care processing part 322 performs a predetermined process corresponding to the input operation.

The TA nurse call processing part 323 performs a voice communication with the sensor unit SU, using the TA sound input and output part 34 and the like. Specifically, the TA nurse call processing part 323 performs a voice communication, for example, by means of VoIP with the sensor unit SU as a sending source having sent the nurse call notification communication signal to the administration server SV, using the TA sound input and output part 34 and the like.

The TA streaming processing part 324 receives a distribution of video images from the sensor unit SU, and causes the TA display part 36 to display the video images received through the distribution by means of the streaming reproduction.

The mobile terminal device TA may be made up by a portable communication terminal device such as a tablet computer, a smart phone and a mobile phone.

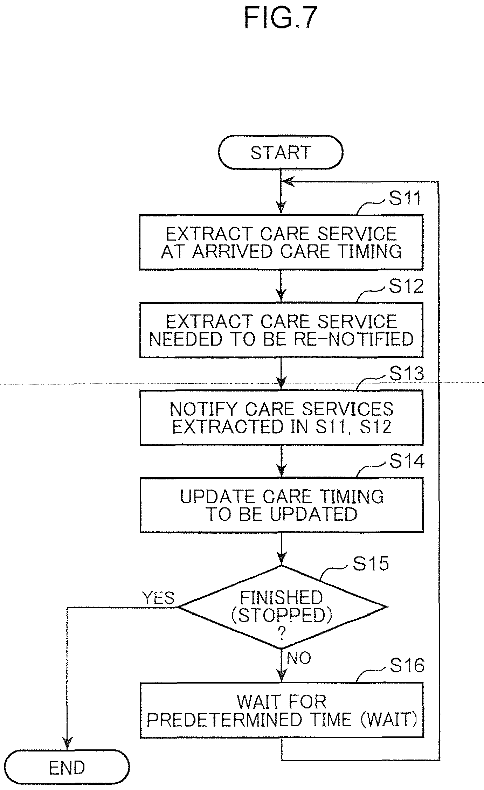

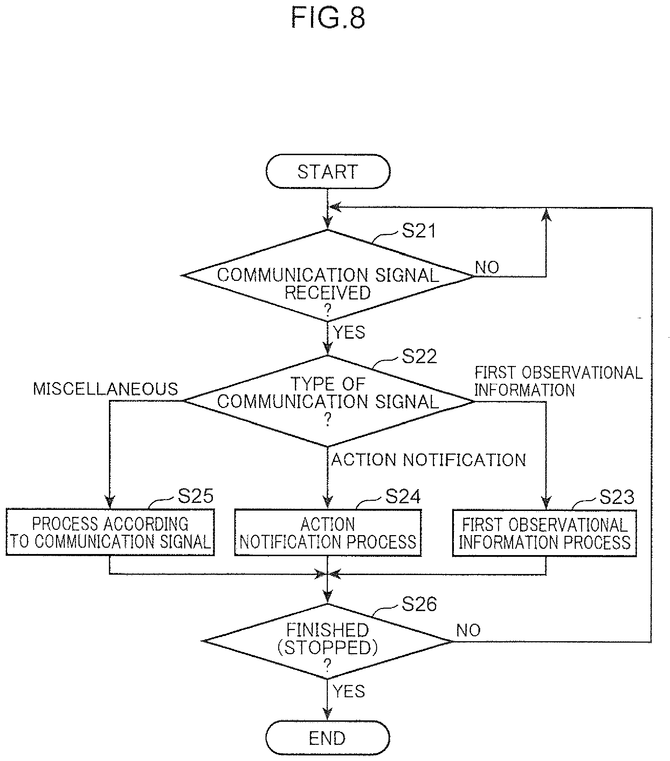

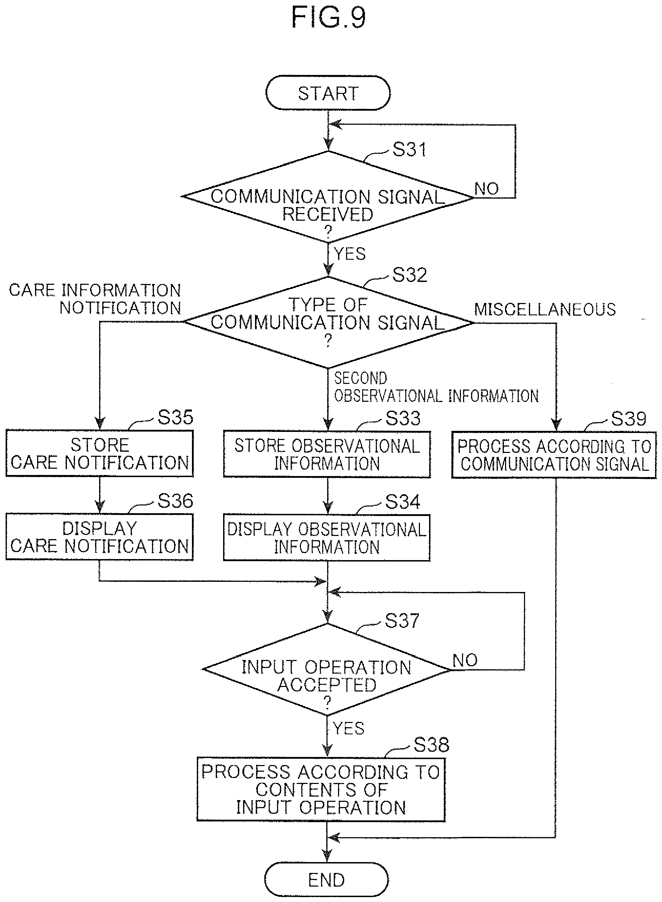

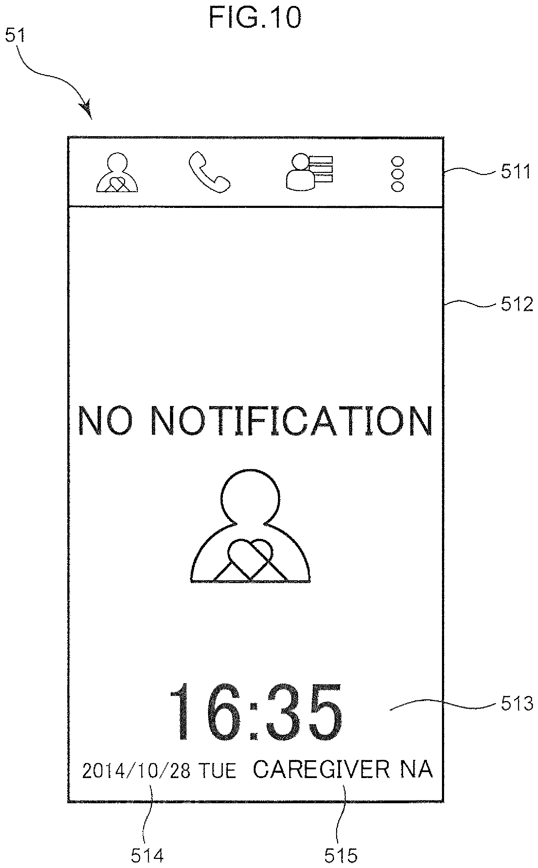

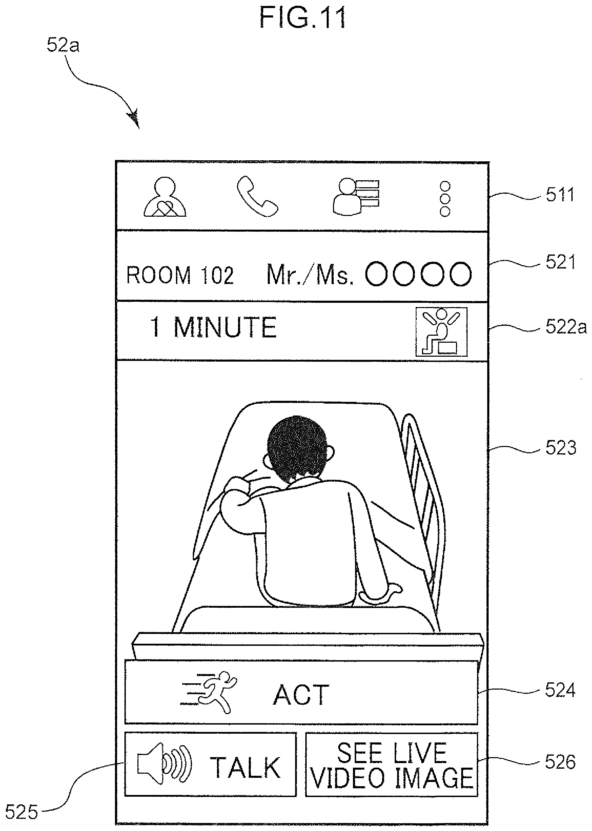

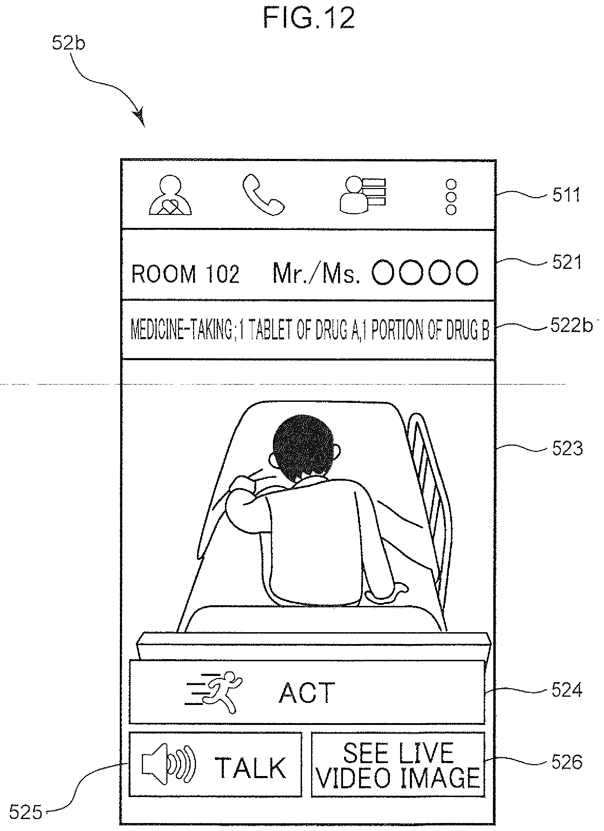

Next, an operation of the embodiment is described. FIG. 7 is a flowchart showing an operation of the mobile terminal device for care notification in the subject observation system of the embodiment. FIG. 8 is a flowchart showing an operation of the administration server for the receipt of a communication signal in the subject observation system of the embodiment. FIG. 9 is a flowchart showing an operation of the mobile terminal device in the subject observation system of the embodiment. FIG. 10 shows an example of a standby screen image displayed on the mobile terminal device in the subject observation system of the embodiment. FIG. 11 shows an example of an observational information screen image displayed on the mobile terminal device in the subject observation system of the embodiment. FIG. 12 shows an example of a care notification screen image displayed on the mobile terminal device in the subject observation system of the embodiment.

In the administration server MS, the respective devices SU, SV, SP, TA initialize respective necessary parts and activate when power is turned on. In the sensor unit SU, the SU control processing section 14 is operably provided with the SU control part 141, the action inspection processing part 142, the SU nurse call processing part 143 and the SU streaming processing part 144 by executing the control processing program thereof. In the administration server SV, the SV control processing section 22 is operably provided with the SV control part 221, the SV observational processing part 222, the care information notification processing part 223, the care timing update processing part 224, the care information storage processing part 225 and the time measuring part 226 by executing the control processing program thereof. In the mobile terminal device TA, the TA control processing section 32 is operably provided with the TA control part 321, the TA observational care processing part 322, the TA nurse call processing part 323 and the TA streaming processing part 324 by executing the control processing program thereof.

First, an operation of the sensor unit SU to inspect a predetermined movement of an subject Ob based on an image and notify the predetermined movement to the predetermined terminal device SP, TA via the administration server SV in the subject observation system MS having the above configuration is described. This operation is roughly performed as follows.

The sensor unit SU generates images of an imaging target at a predetermined sampling cycle by the imaging part 11, performs an inspection process of inspecting the predetermined movement (state, situation) of the subject Ob based on these images by means of the movement inspection processing part 142. As a result, if the predetermined movement (e.g. waking-up, bed-leaving, falling-down, and falling-over movements in the embodiment) of the subject Ob is inspected, the movement inspection processing part 142 generates a first observational information communication signal containing the sensor ID of the sensor unit SU, the inspection result and the images used for the inspection of the predetermined movement, and sends the generated first observational information communication signal to the administration server SV via the SU communication IF part 15.

Upon receipt of the first observational information communication signal from the sensor unit SU via the network NW, the administration server SV stores (records) the observational information about the observation of the subject Ob contained in the received first observational information communication signal in the SV observational information storage processing part 231 by means of the SV observational processing part 222. Then, the SV observational processing part 222 sends a second observational information communication signal containing the sensor ID, the inspection result and the images contained in the first observational information communication signal and a communication address corresponding to the sensor unit SU having the sensor ID contained in the first observational information communication signal as a download destination of video images to the terminal device SP, TA as a transmission destination corresponding to the sensor unit SU having sent the received first observational information communication signal. In this way, the predetermined movement of the subject Ob is notified from the sensor unit SU to the terminal device SP, TA via the administration server SV and further notified to an observer such as a nurse or caregiver via the terminal device SP, TA.

Upon receipt of the second observational information communication signal from the administration server SV, the stationary terminal device SP and the mobile terminal devices TA display the observational information contained in this second observational information communication signal in an observational information screen image 52a, for example, shown in FIG. 11 to be described later. By such an operation, the subject observation system MS roughly inspects each predetermined movement of each subject Ob and observes each subject Ob by means of each sensor unit SU, the administration server SV, the stationary terminal device SP and each mobile terminal device TA.

Next, an operation of notifying the care service in the subject observation system MS is described. First, an operation of the administration server SV is described. Here, it is assumed in the following description that the care information is input using an unillustrated care information input screen image from the stationary terminal device SP or using an unillustrated care information input screen image from the mobile terminal device TA and the input care information is stored in the care information storage part 233 of the administration server SV.

When being activated, the administration server SV starts an operation for the notification of the care service. The administration server SV first extracts the care service whose care timing has arrived by means of the care information notification processing part 223 of the SV control processing section 22 in FIG. 7 (S11). Specifically, the care information notification processing part 223 reads a current time from the time measuring part 226, selects (searches) a record, in which "0" is registered as the notification flag in the notification flag field 2335, out of records having times before the read current time registered as care timings in the care timing field 2331 in the care information table CT stored in the care information storage part 233, and reads the care name, the sensor ID and the supplementary information respectively from the care name field 2332, the sensor ID field 2334 and the supplementary information field 2333 in the selected record. For example, if the current time is 08:32, a record in the fourth row is selected, "excretion assistance", "SU-1" and "*" are read respectively as the care name, the sensor ID and the supplementary information from the care name field 2332, the sensor ID field 2334 and the supplementary information field 2333 in the selected record by the care information notification processing part 223 in an example shown in FIG. 4.

Subsequently, the administration server SV extracts the care service needed to be re-notified by the care information notification processing part 223 of the SV control processing section 22 (S12). Specifically, the care information notification processing part 223 selects (searches) a record, in which "1" is registered as the notification flag in the notification flag field 2335 and "0" is registered as the action flag in the action flag field 2336, out of records having times before the current time read in step S11 registered as care timings in the care timing field 2331 in the care information table CT stored in the care information storage part 233, and reads the care name, the sensor ID and the supplementary information respectively from the care name field 2332, the sensor ID field 2334 and the supplementary information field 2333 in the selected record. For example, if the current time is 08:32, a record in the third row are selected, "medicine-taking", "SU-2" and "one tablet of drug A, 1 portion of drug B" are read respectively as the care name, the sensor ID and the supplementary information from the care name field 2332, the sensor ID field 2334 and the supplementary information field 2333 in the selected record by the care information notification processing part 223 in the example shown in FIG. 4.

Subsequently, the administration server SV notifies the care services extracted respectively in steps S11 and S12 by means of the care information notification processing part 223 (S13). Specifically, the care information notification processing part 223 selects a transmission destination corresponding to the sensor ID read in step S11 from the transmission destination associative relationship stored in the inter-device information storage part 232, acquires an image from the sensor unit SU having the sensor ID read in step S11, sends a care information notification communication signal to the selected terminal device S P, TA, and updates the notification flag, the action flag and the update flag respectively by registering "1", "0" and "0" respectively in the notification flag field 2335, the action flag field 2336 and the update flag field 2338 in the selected record. Such a process is performed for each of all the care services extracted in step S11. Then, the care information notification processing part 223 selects a transmission destination corresponding to the sensor ID read in step S12 from the transmission destination associative relationship stored in the inter-device information storage part 232, acquires an image from the sensor unit SU having the sensor ID read in step S12 and sends a care information notification communication signal to the selected terminal device SP, TA. Such a process is performed for each of all the care services extracted in step S12. If there are a plurality of communication signals having a common sending destination of the care information notification communication signals for the respective care services extracted in respective steps S11 and S12, the plurality of communication signals may be left as they are or may be compiled into one communication signal (data of the plurality of communication signals is aggregated and contained in the one communication signal).

Subsequently, the administration server SV updates the care timing to be updated by means of the care timing update processing part 224 of the SV control processing section 22 (S14). Specifically, if an update timing is set at AM8:00 when the next care timing information is "fixed time", the care timing update processing part 224 determines whether or not the current time read in step S11 is within a predetermined time (standby time) waited (wait process) in step S16 to be described later from AM8:00 as an update timing. If the current time is not within the standby time as a result of this determination, the care timing update processing part 224 selects (searches) a record, in which "1" is registered as the notification flag in the notification flag field 2335, "1" is registered as the action flag in the action flag field 2336 and "0" is registered as the update flag in the update flag field 2338, out of records having times before the current time read in step S11 registered as care timings in the care timing field 2331 in the care information table CT stored in the care information storage part 233, and reads the care timing and the next care timing information respectively from the care timing field 2331 and the next care timing information field 2337 in the selected record. Then, if the read next care timing information represents another update method excluding "fixed time" such as "time interval", the care timing update processing part 224 obtains the next care timing according to the read next care timing information, updates the care timing by registering the obtained next care timing in the care timing field 2331 in the selected record, and updates each of the notification flag, the action flag and the update flag by registering "0", "0" and "1" respectively in the notification flag field 2335, the action flag field 2336 and the update flag field 2338 in the selected record. For example, in an unillustrated case where "7:00" is registered as the care timing in the care timing field 2331 and "after six hours" is registered as the next care timing in the next care timing information field 2337, "13:00" which is obtained six hours after "7:00" is obtained and registered in the care timing field 2331 to update the care timing. It should be noted that although the next care timing is obtained using the next care timing information on the basis of the care timing registered in the care timing field 2331 above, the next care timing may be obtained using the next care timing information on the basis of the current time read in step S11. In this way, the next care timing can be more properly updated if the care service is re-notified. On the other hand, if the current time is within the standby time as a result of the determination, the care timing update processing part 224 selects (searches) a record, in which "1" is registered as the notification flag in the notification flag field 2335, "1" is registered as the action flag in the action flag field 2336 and "0" is registered as the update flag in the update flag field 2338, out of records having "fixed time" registered in the care timing field 2331 in the care information table CT stored in the care information storage part 233, and updates each of the notification flag, the action flag and the update flag by registering "0", "0" and "1" respectively in the notification flag field 2335, the action flag field 2336 and the update flag field 2338 in the selected record.

Subsequently, the administration server SV determines whether or not the activation thereof has been finished (stopped) by means of the SV control processing section 22 (S15). The administration server SV finishes the process if the activation has been finished (stopped) as a result of the determination (Yes) while performing next step S16 if the activation has not been finished (stopped) (No).

In step S16, the administration server SV waits for a predetermined time (wait process) by means of the SV control processing section 22 and, thereafter, returns the process to step S11. The predetermined time is a repeat interval of repeating the respective processes in steps S11 to S15 described above and a time interval for notifying the care service, and is set at an appropriate time such as 3 minutes, 5 minutes or 10 minutes.

The administration server SV performs a process for a communication signal received from the network NW shown in FIG. 8 to be described next while repeatedly performing the operation shown in FIG. 7 described above. In this process, in FIG. 8, the administration server SV first determines whether or not a communication signal has been received by the SV communication IF section 21 by means of the SV control part 221 of the SV control processing section 22 (S21). The administration server SV returns the process to S21 if no communication signal has been received as a result of the determination (No) while performing next step S22 if the communication signal has been received as a result of the determination (Yes). Specifically, the administration server SV waits for the receipt of the communication signal.

In step S22, the administration server SV determines the type of the received communication signal by means of the SV control part 221. Step S26 is performed after step S23 is performed if the received communication signal is a first observational information communication signal (first observational information), step S26 is performed after step S24 is performed if the received communication signal is an action notification communication signal (action notification), and step S26 is performed after step S25 is performed if the received communication signal is neither the first observational information communication signal nor the action notification communication signal (miscellaneous). It should be noted that the sending of the action notification communication signal by the mobile terminal device TA is described later using FIG. 9.

In step S23, the administration server SV processes the first observational information communication signal received from the sensor unit SU in step S21 by means of the SV observational processing part 222 of the SV control processing section 22. Specifically, as described above, the SV observational processing part 222 stores (records) the observational information about the observation of the subject Ob contained in the first observational information communication signal received in step S21 in the SV observational information storage part 231. The SV observational processing part 222 sends a second observational information communication signal containing a sensor ID, an inspection result and images contained in the first observational information communication signal and a communication address corresponding to the sensor unit SU having the sensor ID contained in the first observational information communication signal as a download destination of video images to the terminal device SP, TA as a transmission destination corresponding to the sensor unit SU having sent the received first observational information communication signal.

In step S24, the administration server SV processes the action notification communication signal received from the terminal device SP, TA in step S21 by means of the SV observational processing part 222 of the SV control processing section 22. Specifically, upon receipt of the action notification communication signal from the terminal device SP, TA in step S21, the SV care information storage processing part 225 reads a current time from the time measuring part 226, selects a record, in which the care name and the sensor ID contained in the received action notification communication signal are respectively registered in the care name field 2332 and the sensor ID field 2334, "1" is registered as the notification flag in the notification flag field 2335 and "0" is registered as the action flag in the action flag field 2336, out of records having times before the read current time registered as care timings in the care timing field 2331 in the care information table CT stored in the care information storage part 233, and updates the action flag by registering "1" in the action flag field 236 in the selected record.

In step S25, the administration server SV performs an appropriate process corresponding to the communication signal received in step S21 by means of the SV control processing section 22.

In step S26 performed after each of steps S23 to S25, the administration server SV determines whether or not the activation thereof has been finished (stopped) by means of the SV control processing section 22. The administration server SV finishes the process if the activation has been finished (stopped) as a result of the determination (Yes) while returning the process to step S21 if the activation has not been finished (stopped) (No).

The administration server SV operates as just described.

Next, operations of the terminal devices SP, TA are described. Here, the operation of the mobile terminal device TA is described as a representative. As described above, when the activation is started, the mobile terminal device TA accepts a log-in operation by an observer (user) such as a nurse or a caregiver, the TA observational care processing part 322 renders the TA display part 36 to display a standby screen image indicating a standby for receiving a communication signal directed to the mobile terminal device TA. As shown in FIG. 10, a standby screen image 51 includes, for example, a menu bar region 511 for displaying a menu bar, a standby main region 512 for displaying a message (for example, "no notification") representing the standby and an icon, a time region 513 for displaying a current time, a calendar region 514 for displaying a day of the week, a date, a month, and a year of today, and a user name region 515 for displaying a name of a user who is logging in the mobile terminal device TA.

In FIG. 9, the mobile terminal device TA determines whether or not a communication signal has been received by the TA communication IF section 31 by means of the TA control part 321 of the TA control processing section 32 (S31). The mobile terminal device TA returns the process to S31 if no communication signal has been received as a result of the determination (No) while performing next step S32 if the communication signal has been received as a result of the determination (Yes). Specifically, the mobile terminal device TA waits for the receipt of the communication signal.

In step S32, the mobile terminal device TA determines the type of the received communication signal by means of the TA control part 321. Step S37 is performed after steps S33 and S34 are successively performed if the received communication signal is a second observational information communication signal (second observational information), step S37 is performed after steps S35 and S36 are successively performed if the received communication signal is a care information notification communication signal (care information notification), and the process is finished after step S39 is performed for an appropriate process corresponding to the communication signal received in step S31 if the received communication signal is neither the second observational information communication signal nor the care information notification communication signal (miscellaneous).

In step S33, the TA observational care processing part 322 stores (records) the observational information about the observation of the subject Ob in the TA observational information storage part 331 based on each information (each data) contained in the second observational information communication signal received in step S31.

Subsequently, in step S34, the TA observational care processing part 322 renders the TA display part 36 to display a screen corresponding to each information contained in the received second observational information communication signal, for example, the observational information screen image 52a shown in FIG. 11.

The observational information screen image 52a is a screen image for displaying the observational information about the observation of the subject Ob. As shown in FIG. 11, the observational information screen image 52a includes, for example, a menu bar region 511, a subject name region 521 for displaying an arrangement location of a sensor unit SU having a sensor ID contained in the second observational information communication signal received in step S31 and a name of a subject Ob observed by the sensor unit SU having the sensor ID, an inspection information display region 522a for displaying an elapsed time from a receipt time of the second observational information communication signal received in step S31 (inspection time of the predetermined movement) and an inspection result contained in the second observational information communication signal received in step S31, an image region 523 for displaying an image contained in the second observational information communication signal received in step S31 (i.e. image imaged by the sensor unit SU having the sensor ID) (here, still image), an "act" button 524, a "talk" button 525 and a "see LIVE video image" button 526.

Although the inspection result (name of each of waking-up, bed-leaving, falling-down and falling-over movements in the embodiment) contained in the second observational information communication signal received in step S31 may be displayed as it is in the inspection information display region 522a, an icon symbolically representing the inspection result is displayed in the embodiment. To display the icon, the respective movements and icons symbolically representing the movements are stored in advance in the TA storage section 33 while being associated with each other. In an example shown in FIG. 11, a waking-up icon symbolically representing the waking-up movement is displayed in the inspection information display region 522a. In the observational information screen image 52a, the "act" button 524 is used to input execution intention information representing that the user of the mobile terminal device TA has an intention to perform a predetermined action (reply, execution) such as lifesaving, nursing, care and help in response to the inspection result displayed in the observational information screen image 52a. The "talk" button 525 is used to input a request for a voice communication, and to input an instruction of requesting communicative connection between the sensor unit SU having the sensor ID and the mobile terminal device TA via the network NW. The "see LIVE video image" button 526 is used to input a request for a live video image and to input an instruction of displaying a video image imaged by the sensor unit SU having the sensor ID.

On the other hand, in step S35, the mobile terminal device TA stores (records) care notification information about the care service for the subject Ob contained in the care information notification communication signal received from the administration server SV in step S31 in the care notification information storage part 332 by means of the TA observational care processing part 322 of the TA control processing section 32.

Subsequently, in step S36, the TA observational care processing part 322 renders the TA display part 36 to display a screen image corresponding to each information contained in the received second observational information communication signal, for example, a care notification information screen image 52b shown in FIG. 12.

The care notification information screen image 52b is a screen image for displaying the care notification information about the care service of the subject Ob. As shown in FIG. 12, the care notification information screen image 52b is similar to the observational information screen image 52a shown in FIG. 11 described above, and includes, for example, a menu bar region 511, a subject name region 521 for displaying an arrangement location of a sensor unit SU having a sensor ID contained in the care information notification communication signal received in step S31 and a name of a subject Ob to be observed by the sensor unit SU having the sensor ID, a care information display region 522b for displaying a care name and supplementary information contained in the care information notification communication signal received in step S31, an image region 523 for displaying an image contained in the care information notification communication signal received in step S31 (i.e. image imaged by the sensor unit SU having the sensor ID) (here, still image), an "act" button 524, a "talk" button 525 and a "see LIVE video image" button 526. Specifically, the care notification information screen image 52b shown in FIG. 12 includes the care information display region 522b in place of the inspection information display region 522a of the observational information screen image 52a shown in FIG. 11. It should be noted that an elapsed time from a receipt time of the care information notification communication signal received in step S31 may be further displayed in the care information display region 522b. Here, in the care notification information screen image 52b, the "act" button 524 is used to input execution intention information representing that the user of the mobile terminal device TA has an intention to perform the care service displayed in the care notification information screen image 52b to the mobile terminal device TA.

Referring back to FIG. 9, in step S37, the mobile terminal device TA determines whether or not an input operation has been accepted on the touch panel constituted by the TA input part 35 and the TA display part 36 by means of the TA control processing section 32. The mobile terminal device TA returns the process to step S37 if the input operation has not been accepted as a result of the determination (No) while performing next step S38 if the input operation has been accepted as a result of the determination.

In step S38, the mobile terminal device TA performs an appropriate process corresponding to contents of the input operation by means of the TA control processing section 32 and finishes the screen display operation.

In the mobile terminal device TA, upon acceptance of an input operation of the "act" button 524 (that is, acceptance of the execution intention information) in the observational information screen image 52a, the TA control processing section 32 adds the execution intention information to the observational information of the subject Ob being displayed on the TA display part 36, stores the resultant information in the TA observational information storage part 331, and sends a communication signal (action notification communication signal) containing the sensor ID and the execution intention information corresponding to the observational information of the subject Ob displayed on the TA display part 36 to the administration server SV. Further, upon acceptance of an input operation of the "act" button 524 (that is, acceptance of the execution intention information) in the care notification information screen image 52b, the TA control processing section 32 adds the execution intention information to the care notification information of the care service being displayed on the TA display part 36, stores the resultant information in the TA care notification information storage part 332, and sends a communication signal (action notification communication signal) containing the care name of the care service displayed on the TA display part 36, the sensor ID and the execution intention information to the administration server SV. In the administration server SV having received the action notification communication signal, step S24 is performed as described using FIG. 8. It should be noted that, in order to make it known to the terminal devices SP, TA of the subject observation system MS that the execution intention information is added for the subject Ob corresponding to the sensor unit SU having the sensor ID, the administration server SV may send a communication signal (action acceptance known communication signal) containing the sensor ID and the execution intention information contained in the received action notification communication signal to the terminal devices SP, TA through broadcasting by means of the SV control processing section 22.

For example, in the mobile terminal device TA, upon acceptance of an input operation of the "talk" button 525, the TA nurse call processing part 323 allows a communication signal (talking request communication signal) containing information including a request for a voice communication to be sent to the sensor unit SU observing the subject Ob being displayed on the TA display part 36, whereby the mobile terminal device TA is voice-communicatively connected with the sensor unit SU having responded to the communication signal via the network NW. In this way, a voice communication is possible between the mobile terminal device TA and the sensor unit SU. It should be noted that, in the mobile terminal device TA, upon acceptance of an input operation of an unillustrated "finish" button to input an instruction of finishing the voice communication, the TA nurse call processing part 323 allows a communication signal (talk finish communication signal) containing information including a request for finish of the voice communication to be sent to the sensor unit SU observing the subject Ob being displayed on the TA display part 36. In this way, the voice communication between the mobile terminal device TA and the sensor unit SU is finished.

For example, in the mobile terminal device TA, upon acceptance of an input operation of the "see LIVE video image" button 526, the TA streaming processing part 324 allows a communication signal (video image distribution request communication signal) containing information including a request for a distribution of a live video image to be sent to the sensor unit SU observing the subject Ob being displayed on the TA display part 36, connects the mobile terminal device TA with the sensor unit SU having responded to the communication signal via the network NW to thereby permit downloading of the video image, receives a distribution of the live video image from the sensor unit SU, and renders the TA display part 36 to display the video image received through the distribution by means of streaming reproduction. The video image is displayed in the image region 523 in the observational information screen image 52a and the care notification information screen image 52b for displaying the live video image, and an unillustrated "finish LIVE video image" button is displayed in place of the "see Live video image" button 526. In this way, the live video image is displayed in the mobile terminal device TA. The unillustrated "finish LIVE video image" button is used to request for finish of the video image and to input an instruction of finishing (suspending) the distribution of the video image imaged by the sensor unit SU having the sensor ID to thereby finish (suspend) the display. In the mobile terminal device TA, upon acceptance of an input operation of the "finish LIVE video image" button, the TA streaming processing part 324 allows a communication signal (video image distribution finish communication signal) containing information including the request for finish of the distribution of the video image to be sent to the sensor unit SU observing the subject Ob being displayed on the TA display part 36, and renders the TA display part 36 to display a still image. In this way, the mobile terminal device TA finishes the display of the live video image.

As described above, in the subject observation system MS, the administration server SV as an example of the central processing device of the subject observation system MS and a central processing method for use in the system in the embodiment, not only the care name of the care service, but also an image acquired from the sensor unit SU having the sensor ID associated with the care timing of the care service, i.e. an image showing a state of the subject Ob as a target who receives the care service having the care name is notified to the predetermined terminal device SP, TA by means of the care information notification processing part 223. Thus, the user (observer) of the predetermined terminal device SP, TA can recognize the care timing of the care service by the predetermined terminal device SP, TA, can recognize the state of the subject Ob as a target who receives the care service having the care name by referring to the image by means of the predetermined terminal device SP, TA, and can judge whether or not the notified care service is executable to the subject Ob before the care service having the care name is performed. Thus, the subject observation system MS, the administration server SV and the central processing method can promote the observer to perform a suitable care service at an appropriate time, wherefore more efficient care services can be assisted (supported) for the subject.

Since the subject observation system MS, the administration server SV and the central processing method notify the supplementary information about the care service together with the care name and the image, the care service can be more properly performed.

The subject observation system MS, the administration server SV and the central processing method can automatically update the next care timing by means of the care timing update processing part 224 and can save time and labor for storing a care name and a care timing, for example, for a regular care service in the care information storage part 233, wherefore more efficient care services can be assisted (supported) for the subject.

Since the subject observation system MS, the administration server SV and the central processing method re-notify the care service having no execution intention information by means of the care information notification processing part 223, it is possible to prevent the care service from being not performed and the care service can be more properly performed.

It should be noted that although each care information of all the sensor IDs (each care information of all the sensor units SU), i.e. each care information of all the subjects Ob is compiled into one care information table CT and collectively registered and stored in the care information storage part 233 in the above embodiment, a care information table CT-n may be individually provided for each of all the sensor IDs (for each of all the sensor units SU), i.e. for each of all the subjects Ob, and the care information of each sensor ID (care information of each sensor unit SU), i.e. the care information of each subject Ob may be individually registered and stored in the corresponding care information table. In this case, each step S11 to S16 described using FIG. 7 is performed for each of these individually provided care information tables CT-n.

Further, although the care information notification communication signal is notified (sent) to the terminal device SP, TA having the terminal ID corresponding to the sensor ID associated with the care timing in the above embodiment, this signal may be broadcast or multi-cast.

Further, although the unacted care service is re-notified at a predetermined time interval in step S16 as described using FIG. 7 in the above embodiment, the observer (user) may manually set (input) re-notification and the unacted care service may be re-notified according to the setting. In this case, a "later" button (not shown) for setting re-notification is provided, for example, in the care notification information screen image 52b described using FIG. 12. Upon acceptance of an input operation of this "later" button, the care service is re-notified after the elapse of a predetermined time (e.g. 5 or 10 minutes) set in advance.

Various aspects of technologies are disclosed in this specification as described above. Main technologies among them will be summarized below.

A central processing device according to one aspect is a central processing device for use in a subject observation system including a sensor unit, a central processing device and a terminal device, the sensor unit being configured to inspect a predetermined movement of a subject as a watching target based on an image and notify the movement to the terminal device via the central processing device, the central processing device including a care information storage part for storing a care name indicating a name of a care service, a care timing indicating a timing of performing the care service and a sensor identifier possessed by the sensor unit corresponding to the subject as a target who receives the care service in mutual association, an image acquiring part for acquiring an image from the sensor unit, and a care information notification processing part for notifying a care name associated with a care timing together with an image acquired by the image acquiring part from a sensor unit having the sensor identifier associated with the care timing to a predetermined terminal device in accordance with the care timing stored in the care information storage part. Preferably, the above central processing device further includes a sensor information storage part for storing the sensor identifier and a subject name of the subject in mutual association, the care information notification processing part notifies the care name associated with the care timing and the subject name associated with the sensor identifier stored in the sensor information storage part together with an image acquired by the image acquiring part from the sensor unit having the sensor identifier associated with the care timing in accordance with the care timing stored in the care information storage part. Preferably, in the above central processing device, the care service is at least one of meal assistance for assisting a meal, dressing assistance for assisting dressing (e.g. washing, cleaning changing of clothes), body position conversion for converting a body position, water intake for taking water, medicine-taking for taking medicine, excretion assistance for assisting excretion, hair washing for washing hair, bathing assistance for assisting bathing, infusion exchange for exchanging an infusion and blood drawing for drawing blood.

The central processing device for use in the subject observation system notifies not only the care name, but also an image acquired by the image acquiring part from the sensor unit having the sensor identifier associated with the care timing, i.e. an image showing a state of the subject as a target who receives the care service having the care name, to the predetermined terminal device by means of the care information notification processing part. Thus, a user (observer) of the predetermined terminal device can recognize the care timing of the care service by means of the predetermined terminal device, can recognize the state of the subject as a target who receives the care service having the care name by referring to the image by means of the predetermined terminal device, and can judge whether or not the notified care service is executable to the subject before the care service having the care name is performed. Therefore, the central processing device for use in the subject observation system can promote the observer to perform a suitable care service at an appropriate time and can assist (support) more efficient care services for the subject.

In a central processing device for use in a subject observation system according to another aspect, the care information storage part further stores predetermined supplementary information used in performing the care service in association with the care timing of the care service, and the care information notification processing part notifies the care name and the supplementary information associated with the care timing together with the image acquired by the image acquiring part from the sensor unit having the sensor identifier associated with the care timing to the predetermined terminal device in accordance with the care timing stored in the care information storage part. Preferably, in the above central processing device, the supplementary information is at least one of a procedure of the care service, cautions to be taken in performing the care service, an amount of water when the care service is water intake, the type and amount of medicine and a minimum medication interval when the care service is medicine-taking, the type of liquid and the type and amount of medicine to be added to an infusion when the care service is an infusion exchange, and blood drawing items when the care service is blood drawing.

Since the central processing device for use in the subject observation system also notifies the predetermined supplementary information together with the care name and the image, the care service can be more properly performed.

A central processing device for use in a subject observation system according to another aspect further includes a care timing update processing part for updating the care timing stored in the care information storage part to a next care timing when the care name is notified together with the image to the predetermined terminal device by the care information notification processing part.

The central processing device for use in the subject observation system can automatically update the next care timing and can save time and labor for storing a care name and a care timing, for example, for a regular care service in the care information storage part, wherefore more efficient care services can be assisted (supported) for the subject.

A central processing device for use in a subject observation system according to still another aspect further includes a care information storage processing part for further storing received execution intension information further in association with the care name in the care information storage part upon receipt of the execution intension information representing an intention to perform the care service corresponding to the care name notified from the central processing device from the terminal device, and the care information notification processing part further notifies the care name together with an image acquired by the image acquiring part from the sensor unit having the sensor identifier corresponding to the care name via the care timing to the terminal device if the execution intension information is not associated with the care name after the elapse of a predetermined time after the care name is notified together with the image to the terminal device.

Since the central processing device for use in the subject observation system re-notifies the care service having no execution intension information, it is possible to prevent the care service from being not performed and the care service can be more properly performed.

A central processing method according to further another aspect is a central processing method for use in a subject observation system including a sensor unit, a central processing device and a terminal device, the sensor unit being configured to inspect a predetermined movement of a subject as a watching subject based on an image and notify the movement to the terminal device via the central processing device, the method including a care information storage step of storing a care name indicating a name of a care service, a care timing indicating a timing of performing the care service and a sensor identifier possessed by the sensor unit corresponding to the subject as a target who receives the care service in mutual association in a care information storage part, an image acquiring step of acquiring an image from the sensor unit, and a care information notification processing step of notifying a care name associated with a care timing together with an image acquired from the sensor unit having the sensor identifier associated with the care timing in the image acquiring step to a predetermined terminal device in accordance with the care timing stored in the care information storage part.

The central processing method for use in the subject observation system notifies not only the care name, but also the image acquired from the sensor unit having the sensor identifier associated with the care timing in the image acquiring step, i.e. an image showing a state of the subject as a target who receives the care service having the care name, to the predetermined terminal device in the care information notification processing step. Thus, a user (observer) of the predetermined terminal device can recognize the care timing of the care service by means of the predetermined terminal device, can recognize the state of the subject as a target who receives the care service having the care name by referring to the image by means of the predetermined terminal device, and can judge whether or not the notified care service is executable to the subject. Therefore, the central processing method for use in the subject observation system can promote the observer to perform a suitable care service at an appropriate time and can assist (support) more efficient care services for the subject.

A subject observation system according to another aspect includes a sensor unit, a central processing device and a terminal device, the sensor unit being configured to inspect a predetermined movement of a subject as a watching subject based on an image and notify the movement to the terminal device via the central processing device, the central processing device being any one of the central processing devices described above.

Since the subject observation system uses any one of the central processing devices described above, an observer can be promoted to perform a suitable care service at an appropriate time and can assist (support) more efficient care services for the subject.

This application is based on Japanese patent application No. 2015-239917 filed in Japan Patent Office on December 9, 2015, the contents of which are hereby incorporated by reference.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

### Industrial Applicability

The present invention can provide a central processing device and a central processing method for use in a subject observation system, and a subject observation system.

## Claims

1. A central processing device for use in a subject observation system including a sensor unit, a central processing device and a terminal device, the sensor unit being configured to inspect a predetermined movement of a subject and notify the movement to the terminal device via the central processing device, comprising:
a care information storage part which stores a care name indicating a name of a care service, a care timing indicating a timing of performing the care service and a sensor identifier possessed by the sensor unit corresponding to the subject as a target who receives the care service in mutual association;
an image acquiring part which acquires an image from the sensor unit; and
a care information notification processing part which notifies a care name associated with a care timing together with an image acquired by the image acquiring part from a sensor unit having the sensor identifier associated with the care timing to a predetermined terminal device in accordance with the care timing stored in the care information storage part.

2. A central processing device according to claim 1, wherein:
the care information storage part further stores predetermined supplementary information used in performing the care service further in association with the care timing of the care service; and
the care information notification processing part notifies the care name and the supplementary information associated with the care timing together with the image acquired by image acquiring part from the sensor unit having the sensor identifier associated with the care timing to the predetermined terminal device in accordance with the care timing stored in the care information storage part.

3. A central processing device according to claim 1 or 2, further comprising:
a care timing update processing part which updates the care timing stored in the care information storage part to a next care timing when the care name is notified together with the image to the predetermined terminal device by the care information notification processing part.

4. A central processing device according to any one of claims 1 to 3, further comprising:
a care information storage processing part which further stores received execution intension information further in association with the care name in the care information storage part upon receipt of the execution intension information representing an intention to perform the care service corresponding to the care name notified from the central processing device from the terminal device;
wherein the care information notification processing part further notifies the care name together with an image acquired by the image acquiring part from the sensor unit having the sensor identifier corresponding to the care name via the care timing to the predetermined terminal device if the execution intension information is not associated with the care name after the elapse of a predetermined time after the care name is notified together with the image to the terminal device.

5. A central processing method for use in a subject observation system including a sensor unit, a central processing device and a terminal device, the sensor unit being configured to inspect a predetermined movement of a subject based on an image and notify the movement to the terminal device via the central processing device, comprising:
a care information storage step of storing a care name indicating a name of a care service, a care timing indicating a timing of performing the care service and a sensor identifier possessed by the sensor unit corresponding to the subject as a target who receives the care service in mutual association in a care information storage part;
an image acquiring step of acquiring an image from the sensor unit; and
a care information notification processing step of notifying a care name associated with a care timing together with an image acquired from the sensor unit having the sensor identifier associated with the care timing in the image acquiring step to a predetermined terminal device in accordance with the care timing stored in the care information storage part.

6. A subject observation system including a sensor unit, a central processing device and a terminal device, the sensor unit being configured to inspect a predetermined movement of a subject based on an image and notify the movement to the terminal device via the central processing device,
wherein the central processing device is the central processing device according to any one of claims 1 to 4.
